(19) Europäisches Patentamt
European Patent Office
Office européen des brevets

(11) **EP 4 153 254 B1**

(12) **EUROPÄISCHE PATENTSCHRIFT**

(45) Veröffentlichungstag und Bekanntmachung des Hinweises auf die Patenterteilung:
**18.06.2025   Patentblatt 2025/25**

(21) Anmeldenummer: **20727223.8**

(22) Anmeldetag: **18.05.2020**

(51) Internationale Patentklassifikation (IPC):
*A61L 9/012* (2006.01)     *B65D 51/24* (2006.01)
*B65D 47/26* (2006.01)     *A47G 21/18* (2006.01)
*B65D 51/28* (2006.01)     *A61L 9/12* (2006.01)

(52) Gemeinsame Patentklassifikation (CPC):
**A61L 9/012; A47G 19/2205; A47G 19/2272; A61L 9/12; B65D 51/2857; B65D 51/2892;** A47G 2400/04; A61L 2209/133; A61L 2209/134; A61L 2209/15

(86) Internationale Anmeldenummer:
**PCT/EP2020/063793**

(87) Internationale Veröffentlichungsnummer:
**WO 2021/233516 (25.11.2021 Gazette 2021/47)**

(54) **AROMABEHÄLTER SOWIE TRINKVORRICHTUNG MIT EINEM AROMABEHÄLTER**

AROMA CONTAINER AND DRINKING DEVICE HAVING AN AROMA CONTAINER

RÉCIPIENT D'ARÔME ET DISPOSITIF DE BOISSON AYANT UN RÉCIPIENT D'ARÔME

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(43) Veröffentlichungstag der Anmeldung:
**29.03.2023   Patentblatt 2023/13**

(73) Patentinhaber: **air up group GmbH**
**81671 München (DE)**

(72) Erfinder:
• **JÜNGST, Magdalena**
**81667 München (DE)**
• **KOPPITZ, Jannis**
**80333 München (DE)**
• **JÄGER, Tim**
**81245 München (DE)**
• **SCHLANG, Fabian**
**81925 München (DE)**

(74) Vertreter: **Hoffmann Eitle**
**Patent- und Rechtsanwälte PartmbB**
**Arabellastraße 30**
**81925 München (DE)**

(56) Entgegenhaltungen:
WO-A1-2019/016096     WO-A1-2019/016096
DE-U1- 202018 000 382     DE-U1- 202018 000 382
JP-A- 2008 104 557     JP-A- 2008 104 557
JP-A- 2013 121 556     JP-A- 2013 121 556

**Beschreibung**

Gebiet der Erfindung

Die Erfindung betrifft einen Aromabehälter sowie eine Trinkvorrichtung mit einem derartigen Aromabehälter.

Stand der Technik

Es besteht ein zunehmendes Bedürfnis, Trinkflüssigkeiten aufzunehmen, die einerseits eine angenehme Geschmacksrichtung besitzen, andererseits aber Gesundheitsrisiken zu vermeiden, die durch die Aufnahme von in der Trinkflüssigkeit gelösten Aromasubstanzen oder Stabilisierungsmittel hervorgerufen werden können. Darüber hinaus soll die Aufnahme einer erhöhten Kalorienmenge vermieden werden.

Daher ist Wasser, das mit einem schwachen Fruchtaroma versehen ist, in den vergangenen Jahren populär geworden. Allerdings finden sich auch in diesem aromatisierten Wasser unerwünschte Additive, wie Stabilisierungssubstanzen und ein gewisser Anteil an Zucker. Viele Benutzer lehnen bereits aufgrund dieser Kalorienmenge derartige Getränke ab.

Da der olfaktorische Sinneseindruck einen wesentlichen Teil der gustatorischen Wahrnehmung beim Konsum von Speisen und Getränken ausmacht, versuchen bisherige Trinksysteme den beim Trinken wahrgenommenen Geruch zu beeinflussen. Dazu werden Aromaelemente vorgeschlagen, die an einem Trinkbehälter nahe der Trinköffnung befestigt werden können, damit sich das Aromaelement in unmittelbarer Nähe zur Nase des Benutzers befindet, der während des Trinkens durch die Nase atmet und somit den Duft aufnimmt.

Aus der WO 2019/016096 A1 ist eine Trinkvorrichtung bekannt zur retronasalen Aufnahme einer Aromasubstanz mit einem luftdurchströmbaren Aromabehälter, der aromatisierte Luft einem Transportkanal für Trinkflüssigkeit zuführt. Auf diese Weise wird die Aromasubstanz retronasal aufgenommen. Die Aromasubstanz gelangt beim Trinken gemeinsam mit der Trinkflüssigkeit in den Mund des Benutzers und steigt anschließend retronasal über den Rachenraum zur Riechschleimhaut auf, wo sie durch die dort befindlichen Rezeptoren erfasst und vom Benutzer wahrgenommen wird. Dabei macht man sich den Umstand zunutze, dass ein enger Zusammenhang zwischen dem Geruchssinn und dem Geschmackssinn besteht. Der Benutzer gewinnt daher den Eindruck, als würde er das Aroma schmecken, obwohl er es tatsächlich lediglich retronasal riecht.

Die WO 2019/016096 A1 sowie die DE 20 2018 000 382 U1 beschreiben einen Aromabehälter mit den Merkmalen des Oberbegriffs des Anspruchs 1.

Darstellung der Erfindung

Der Erfindung liegt die Aufgabe zugrunde einen Aromabehälter zur Zugabe einer Aromasubstanz zu einem den Aromabehälter durchströmenden Luftstrom vorzuschlagen, der eine verbesserte Anreicherung der durchströmten Luft mit Aromasubstanz erlaubt.

Diese Aufgabe wird durch einen Aromabehälter mit den Merkmalen des Anspruchs 1 sowie durch eine Trinkvorrichtung mit den Merkmalen des Anspruchs 16 gelöst. Bevorzugte Ausführungsformen folgen aus den übrigen Ansprüchen.

Der erfindungsgemäße Aromabehälter nach Anspruch 1 zur Zugabe einer Aromasubstanz zu einem den Aromabehälter durchströmenden Luftstrom umfasst eine obere Wand, eine untere Wand sowie mindestens eine Seitenwand, die eine Aromakammer umschließen, mindestens eine Lufteintrittsöffnung in die Aromakammer sowie mindestens eine Luftaustrittsöffnung aus der Aromakammer. In der Aromakammer befindet sich eine Trägersubstanz für die Aromasubstanz. Darüber hinaus ist ein Kopfraum zwischen der Trägersubstanz und der der Aromakammer zugewandten Seite der oberen Wand vorgesehen. Weiterhin umfasst die Trägersubstanz erfindungsgemäß ein Vliesmaterial, wobei die Luftdurchlässigkeit L des Vliesmaterials bei 100Pa Differenzdruck L $\geq$ 200 1/(m$^2$·s) beträgt, und bevorzugt zwischen 220 1/(m$^2$·s) und 280 1/(m$^2$·s) beträgt.

In der Aromakammer befindet sich somit eine Trägersubstanz für die Aromasubstanz sowie ein Kopfraum zwischen der Trägersubstanz und der die Aromakammer nach oben abschließenden Wandung. Das Vorsehen eines Kopfraums verbessert die Durchmischung des aromatisierten Luftstroms. Der die Aromakammer durchströmende Luftstrom durchströmt dabei die Trägersubstanz, die mit der Aromasubstanz versehen ist. Dabei bilden sich innerhalb der Trägersubstanz, die ein Vliesmaterial umfasst, unterschiedliche Teilströmungen aus, die insbesondere wenn der Aromabehälter schon länger in Verwendung ist und sich nicht mehr so viel Aromasubstanz in der Trägersubstanz befindet, ungleich stark aromatisiert werden können. Bei der Durchströmung des Kopfraums, der einen nicht mit Trägersubstanz gefüllten Hohlraum innerhalb der Aromakammer darstellt, kommt es zu einer Homogenisierung der möglicherweise unterschiedlich stark aromatisierten Teilluftströme. Das Vorsehen eines Kopfraums stellt somit sicher, dass der Benutzer einer Trinkvorrichtung, der neben Trinkflüssigkeit den aromatisierten Luftstrom aufnimmt, ein möglichst gleichmäßiges Geschmackserlebnis erfährt.

Ein weiterer Vorteil des Kopfraums besteht darin, die Aromatisierung des Luftstroms mit unterschiedlichen Aromasubstanzen zu homogenisieren. Zahlreiche Aromasubstanzen bestehen nicht aus einer einzigen chemischen Verbindung, sondern umfassen eine Vielzahl von chemischen Verbindungen, deren Stoffübergangsverhalten von der Trägersubstanz in den Luftstrom unterschiedlich ist. Einzelne, aus dem Trägermaterial austretende Teilluftströme können daher eine unterschiedliche chemische Zusammensetzung der Aromatisierung besitzen. Durch das Vermischen der Teilluftströme im Kopfraum wird ein Luftstrom erzeugt, der möglichst homogen mit Aromasubstanz mit der gewünschten Zusammensetzung der chemischen Verbindungen versehen ist.

Ein weiterer wichtiger Aspekt besteht darin, dass die Luftdurchlässigkeit des Vliesmaterials bei 100Pa Differenzdruck mehr als 200 1/(m²·s) beträgt. Eine derartige Luftdurchlässigkeit des Vliesmaterials stellt sicher, dass eine gute Durchströmung durch den den Aromabehälter durchströmenden Luftstrom gewährleistet ist, gleichzeitig aber kein zu hoher Druckverlust entsteht, der die Abgabe der aromatisierten Luft in den Transportkanal für Trinkflüssigkeit einer zugehörigen Trinkvorrichtung erschweren würde.

Es ist somit das Zusammenspiel wichtig einer Trägersubstanz umfassend ein Vliesmaterial, das nicht die gesamte Aromakammer ausfüllt, sondern einen Kopfraum belässt, sowie die geeignete Auswahl einer hohen Luftdurchlässigkeit des Vliesmaterials, die nicht unter 200 1/(m²·s) sein sollte.

Vorzugsweise beträgt die Luftdurchlässigkeit L des Vliesmaterials bei 100Pa Differenzdruck nicht mehr als 500 1/(m²·s). Wenn die Luftdurchlässigkeit zu hoch wird, besteht die Gefahr, dass sich innerhalb des Vliesmaterials bevorzugte Strömungspfade bilden, die mit sehr geringem Widerstand von dem den Aromabehälter durchströmenden Luftstrom durchströmt werden können, so dass der Luftstrom nur noch eine geringe Tendenz besitzt, andere Bereiche des Vliesmaterials zu durchströmen und die dort vorhandenen Aromasubstanzen aufzunehmen. Dies würde dazu führen, dass der aus dem Aromabehälter austretende Luftstrom lange bevor die Aromasubstanz tatsächlich erschöpft ist, einen geringer werdenden Aromatisierungsgrad zeigt.

Die Trägersubstanz in der Aromakammer muss nicht dieselbe Erstreckung wie die Geometrie der Aromakammer besitzen. Es kann somit neben dem in vertikaler Richtung zwischen der Trägersubstanz und der oberen Wand vorgesehenen Kopfraum auch in lateraler Richtung im Aromabehälter eine oder mehrere Zonen geben, in denen sich die Trägersubstanz nicht befindet und in denen es zu einer verstärkten Homogenisierung des den Aromabehälter durchströmenden Luftstroms kommen kann.

Wenn bei einer Trinkvorrichtung verschiedene Aromen verwendet werden, kann sich die am besten geeignete Geometrie in Bezug auf die Größe des Kopfraums, aber auch die laterale Erstreckung der Trägersubstanz in der Aromakammer zwischen den einzelnen Aromasubstanzen unterscheiden. Unterschiedliche Aromasubstanzen weisen eine unterschiedliche chemische Struktur auf, die ihr Verhalten in Bezug auf die Stoffübertragung in den Luftstrom beeinflusst.

Wenn die Trägersubstanz nicht die gesamte untere Wand der Aromakammer bedeckt, kann dies auch den Vorteil besitzen, dass bei komplexen Geometrien der Querschnittsfläche der Aromakammer das Einlegen der Trägersubstanz in der Produktion vereinfacht wird.

Die Trägersubstanz liegt bevorzugt auf der der Aromakammer zugewandten Seite der unteren Wand auf.

Nach einer bevorzugten Ausführungsform der Erfindung umfasst die Seitenwand des Aromabehälters zumindest abschnittsweise einen Vorsprung, der sich vorzugsweise im Wesentlichen senkrecht zu äußeren Seitenwand nach außen erstreckt.

Ein solcher Vorsprung erleichtert zum einen die manuelle Handhabung durch einen Benutzer, der neben einer geeigneten Etikettierung anhand des Vorsprungs identifizieren kann, in welcher Orientierung der Aromabehälter mit einer zugehörigen Trinkvorrichtung gekoppelt werden muss. Darüber hinaus kann, wie später erläutert werden wird, ein sich von der Seitenwand nach außen erstreckender Vorsprung dazu benutzt werden, um in Zusammenwirken mit einer entsprechend gestalteten Trinkvorrichtung den Aromabehälter automatisch in eine Betriebsposition zu bewegen und umgekehrt auch in eine Position zu bringen, in welcher der Aromabehälter auch über einen längeren Zeitraum gelagert werden kann, weil ein ungewolltes Ausdünsten des Aromabehälters verhindert wird.

Vorzugsweise besitzt das Vliesmaterial einen spezifischen Strömungswiderstand von weniger als 500 Pa·s/m und bevorzugt unter 400 Pa·s/m und am meisten bevorzugt von etwa 380 Pa·s/m. Dabei wird entsprechend der üblichen Definition das Verhältnis der Druckdifferenz vor und hinter einer Materialschicht zur Geschwindigkeit der durchströmenden Luft als spezifischer Strömungswiderstand bezeichnet.

Weiterhin bevorzugt ist es, dass das Vliesmaterial ein Flächengewicht von unter 1500 g/m² und bevorzugt von etwa 1000 g/m² besitzt und das Vliesmaterial eine Dichte von weniger als 300 kg/m³ und bevorzugt eine Dichte von etwa 200 kg/m³ besitzt.

Das Flächengewicht, die maximale Dichte aber auch der bevorzugte spezifische Strömungswiderstand haben sich dabei als besonders geeignete Kenngrößen gezeigt, um eine gute Durchströmung der Trägersubstanz in der Aromakammer, aber auch eine gute Anreicherung der Luft mit Aroma und eine verbesserte Aromaentfaltung bei der Verwendung des Aromabehälters in einer zugehörigen Trinkvorrichtung zu ermöglichen.

Als Vliesmaterial ist weiterhin 100% Polyester bevorzugt, da es sich dabei um ein inertes Material handelt, das mit den Aromasubstanzen keine unerwünschte Wechselwirkung eingeht.

Weiterhin hat sich als vorteilhaft erwiesen, die Trägersubstanz mit einer Dicke von 2 mm bis 10 mm und bevorzugt von etwa 5mm in der Aromakammer anzuordnen.

Nach einer bevorzugten Ausführungsform der Erfindung beträgt die Dicke der Trägersubstanz mindestens 50% der Höhe zwischen der oberen Wand des Aromabehälters und der unteren Wand des Aromabehälters und bevorzugt mindestens 80% der Höhe zwischen der oberen Wand des Aromabehälters und der unteren Wand des Aromabehälters. Wenn die obere Wand und die untere Wand des Aromabehälters nicht parallel zueinander verlaufen, sind die geometrisch gemittelte Dicke der Trägersubstanz sowie die geometrisch gemittelte Höhe der Aromakammer im Bereich der Trägersubstanz anzusetzen.

Wenn die Dicke der Trägersubstanz weniger als 50% der lichten Höhe innerhalb der Aromakammer beträgt, findet keine ausreichende Durchströmung des Vliesmaterials mehr statt. Um eine gute Durchströmung des Vliesmaterials sicherzustellen, ist daher besonders bevorzugt die Dicke der Trägersubstanz mindestens 80% der Höhe zwischen der oberen Wand des Aromabehälters und der unteren Wand des Aromabehälters.

Eine weitere bevorzugte Ausführungsform sieht vor, dass die Trägersubstanz aus mehr als einem Teil besteht, wie das beispielsweise bei Granulaten der Fall ist. Ebenso kann das Trägermaterial im Wesentlichen längs oder quer bezüglich sowohl der Faserrichtung des Materials als auch bezüglich der Geometrie der Aromakammer mindestens einmal geteilt sein. Dies ermöglicht ein erleichtertes Füllen der Aromakammer mit dem Trägermaterial.

Eine besonders bevorzugte Ausführungsform des Trägermaterials sieht vor, dass dieses bereits vor dem Einlegen in die Aromakammer mit einer Aromasubstanz versetzt ist, oder aber während oder nach dem Einlegen dieser in die Aromakammer mit einer Aromasubstanz versetzt wird. Ebenso kann die gegenständliche Erfindung auch durch den Nutzer selbst befüllt werden oder nachfüllbar gestaltet sein.

Die Höhe zwischen der oberen Wand und der unteren Wand definiert die Höhe des inneren Volumens der Aromakammer. Der Kopfraum über der Trägersubstanz soll folglich im Bereich der Trägersubstanz nicht mehr als die halbe Höhe des Aromabehälters zwischen oberer Wand und unterer Wand betragen.

Nach einer bevorzugten Ausführungsform der Erfindung besitzt die mindestens eine Lufteintrittsöffnung einen Durchmesser von mindestens 0.2 mm oder eine unterschiedliche Geometrie mit äquivalentem minimalen Öffnungsquerschnitt.

Ein geringerer Öffnungsquerschnitt der Lufteintrittsöffnung als 0.2 mm bringt einen zu hohen Druckverlust mit sich und erschwert daher die Durchströmung des Aromabehälters. Darüber hinaus besitzt eine weitere Verringerung des Öffnungsquerschnitts der Lufteintrittsöffnung auch keine Vorteile in Bezug auf das in diesem Bereich herrschende Strömungsregime, das bei üblichen Betriebsbedingungen ohnehin bereits im turbulenten Bereich liegt.

Vorzugsweise liegt die Porosität des Vliesmaterials zwischen 70% und 93% und bevorzugt zwischen 70% und 80%.

Eine hohe Porosität des Vliesmaterials ist nicht nur im Hinblick auf eine gute Durchströmbarkeit und einen geringen spezifischen Strömungswiderstand wichtig, sondern ermöglicht es, dass die Trägersubstanz viel Aromasubstanz aufnehmen kann, so dass ein Aromabehälter mit geringen Abmessungen eine lange Gebrauchsdauer bis zur Erschöpfung des Vorrats an Aromasubstanz besitzt.

Nach einer bevorzugten Ausführungsform der Erfindung ist die Reynoldszahl Re im Lufteintrittsbereich des Aromabehälters größer als 2000, wobei die Reynoldszahl definiert ist als $Re = (w \cdot d)/v$, mit der kinematischen Viskosität von Luft $v$ [m²/s], dem Durchmesser $d$[m] der Lufteintrittsöffnung und der Strömungsgeschwindigkeit $w$[m/s] beim Einströmen in den Aromabehälter mit einem zeitlich gemittelten Volumenstrom zwischen 250 ml/min und 600 ml/min.

Eine Reynoldszahl von größer als etwa 2000 charakterisiert eine turbulente Strömung oder zumindest eine Strömung, die sich bereits im Übergangsbereich zwischen einer laminaren und turbulenten Strömung befindet. Eine turbulente Strömung gewährleistet eine gute Durchmischung, wodurch die Aufnahme der Aromasubstanz in dem den Aromabehälter durchströmenden Luftstrom verbessert wird. Der Volumenstrom pro Minute berücksichtigt die zum Teil erheblichen Schwankungen im temporären Volumenstrom, wenn der erfindungsgemäße Aromabehälter mit einer Trinkvorrichtung gekoppelt ist und die Luftaustrittsöffnung mit einem Transportkanal für Trinkflüssigkeit in Strömungsverbindung steht. Da der Trinkvorgang kein gleichmäßiger, stationärer Vorgang ist, sondern im Rahmen der Saug- und Schluckvorgänge unterschiedliche Drücke, aber auch unterschiedliche Strömungsgeschwindigkeiten im Transportkanal für Trinkflüssigkeit herrschen, besteht eine beträchtliche Fluktuation der temporären Volumenströme und damit auch Strömungsgeschwindigkeiten, mit denen die Luft durch die Lufteintrittsöffnung hindurchtritt. Experimentell konnte jedoch gezeigt werden, dass ein erfindungsgemäßer Aromabehälter bei seinem bestimmungsgemäßen Einsatz mit einem gemittelten Volumenstrom zwischen 250 ml/min und 600 ml/min durchströmt wird. Der physiologische Trinkvorgang wurde dabei an zahlreichen, unterschiedlichen, erwachsenen Personen ermittelt. Da der Volumenstrom das Produkt aus Strömungsgeschwindigkeit und Querschnittsfläche ist, kann sich unter der Vorgabe, dass die den Strömungszustand beschreibende Reynoldszahl größer als 2000 sein muss, ein geeigneter Bereich für den Öffnungsquerschnitt der Lufteintrittsöffnung bestimmen.

Vorzugsweise besitzt die Lufteintrittsöffnung einen Durchmesser von zwischen 0.2 mm und 20 mm. Eine relativ große Lufteintrittsöffnung von 20 mm kann zwar direkt im Bereich der Lufteintrittsöffnung keine turbulente Strömung sicherstellen, doch kann dies durch die geeignete Wahl der Geometrie der Aromakammer kompensiert werden, beispielsweise indem die Trägersubstanz in der Aromakammer direkt an der Lufteintrittsöffnung anliegt und somit der in der Aromakammer eintretende Luftstrom innerhalb des Vliesmaterials die Trägersubstanz turbulent durchströmt.

Nach einer bevorzugten Ausführungsform der Erfindung weist der Aromabehälter eine im Wesentlichen ringförmige Geometrie auf mit einer äußeren Seitenwand und einer inneren Seitenwand.

Eine derartige Geometrie besitzt zahlreiche Vorteile. Zum einen lässt sich ein derartiger Aromabehälter um den Transportkanal für Trinkflüssigkeit am Kopfteil einer Trinkvorrichtung befestigen, ohne dass das Gewicht des Aromabehälters als störend empfunden wird, da es nicht zu einer unsymmetrischen Gewichtsverteilung an der Trinkvorrichtung kommt. Darüber hinaus kann durch das Vorsehen einer ringförmigen Geometrie gezielt durch die Anordnung der Lufteintrittsöffnung und der Luftaustrittsöffnung sichergestellt werden, dass der den Aromabehälter durchströmende Luftstrom einen möglichst langen Weg zurücklegt und dabei die in der Aromakammer enthaltene Trägersubstanz möglichst vollständig durchströmt.

Vorzugsweise umschließt bei einem Aromabehälter mit einer im Wesentlichen ringförmigen Geometrie die innere Seitenwand einen Raum, dessen Querschnittsfläche eine von einer kreisrunden Form abweichende Geometrie besitzt.

Diese Gestaltung hat zahlreiche Vorteile wie anhand mehrerer Beispiele beschrieben werden wird. Zum einen kann gezielt die Reibung zwischen der inneren Seitenwand und einem Kopfteil einer Trinkvorrichtung, auf welche der Aromabehälter aufgesetzt wird, verringert werden. Darüber hinaus kann eine nicht kreisrunde Form dazu verwendet werden, den Aromabehälter im Zusammenwirken mit einer geeignet gestalteten Trinkvorrichtung zwischen unterschiedlichen Betriebspositionen zu bewegen. Schließlich kann eine nicht kreisrunde Form auch dazu verwendet werden, einem Benutzer das korrekte Platzieren des Aromabehälters an einer Trinkvorrichtung zu erleichtern.

In diesem Zusammenhang ist es besonders vorteilhaft, den Aromabehälter so auszugestalten, dass die Geometrie der inneren Seitenwand nur eine einzige Position des Aromabehälters in Bezug auf seine Rotation im Zusammenwirken mit einem entsprechend geformten Mundstück einer Trinkvorrichtung definiert, wobei vorzugsweise der durch die innere Seitenwand umschlossene Raum eine im Wesentlichen tropfenförmige Querschnittsfläche besitzt.

Mit anderen Worten ist die Geometrie der inneren Seitenwand des Aromabehälters so gewählt, dass der durch die innere Seitenwand umschlossene Raum eine Querschnittsfläche besitzt, die eine eindeutige Positionierung des Aromabehälters erlaubt. Eine hexagonale Geometrie der inneren Seitenwand könnte dies beispielsweise nicht leisten. Der von der inneren Seitenwand umschlossene Raum besäße zwar eine von einer kreisrunden Form abweichende Geometrie der Querschnittsfläche, jedoch könnte der Aromabehälter auf einem entsprechend geformten und den inneren Raum füllenden Mundstück in sechs unterschiedlichen Rotationspositionen aufgesetzt werden.

Es hat sich als besonders vorteilhaft gezeigt, wenn bei einer im Wesentlichen ringförmigen Geometrie des Aromabehälters, dessen Querschnittsfläche jedoch von einer kreisrunden Form abweicht, für das Verhältnis zwischen einer maximalen Erstreckung ($L_{max}$) der Querschnittsfläche des von der inneren Seitenwand umschlossenen Raums und der minimalen Erstreckung ($L_{min}$) der Querschnittsfläche des von der inneren Seitenwand umschlossenen Raums gilt:

$$1.05 \leq L_{max}/L_{min} \leq 1.15;$$

und bevorzugt

$$L_{max}/L_{min} \text{ etwa } 1.1$$

beträgt.

Eine von einer kreisrunden Geometrie abweichende Geometrie des im Wesentlichen ringförmigen Aromabehälters hat sich dabei nicht nur in Bezug auf mögliche eindeutige Positionierungsoptionen als vorteilhaft erwiesen, sondern hat auch die Wirkung, dass der den Aromabehälter durchströmende Luftstrom durch Querschnittsänderungen und Änderungen der Krümmung der Aromakammer besser durchmischt wird, weil sämtliche Änderungen im Strömungsverlauf das Auftreten von Turbulenzen unterstützen. Würde das Verhältnis zwischen der minimalen Erstreckung und der maximalen Erstreckung zu groß werden, dann hätte dies hingegen wieder negative Auswirkungen in Bezug auf die von dem Luftstrom zu durchströmende Wegstrecke in Bezug auf das Volumen der Aromakammer. Hier ist das Optimum bei einer reinen Kreisringform erreicht. Die Spanne zwischen Werten von 1.05 und 1.1 für $L_{max}/L_{min}$ stellt somit einen bestmöglichen Kompromiss zwischen einer möglichst langen Wegstrecke des den Aromabehälter durchströmenden Luftstroms einerseits und der Unterstützung des Auftretens von Turbulenzen und/oder der Aufrechterhaltung bestehender Turbulenzen andererseits dar.

Vorzugsweise umfasst der Aromabehälter weiterhin Gleitrippen im Bereich der inneren Seitenwand. Durch das Vorsehen von Gleitrippen wird die Reibung beim Aufschieben des im Wesentlichen ringförmigen Aromabehälters auf eine Trinkvorrichtung verringert, indem die Kontaktfläche zwischen dem Aromabehälter und dem sich in dem durch die innere Seitenwand umschlossenen Raum erstreckenden Mundstück der Trinkvorrichtung reduziert wird.

In gleicher Weise ist es aber auch möglich anstelle des Vorsehens von Gleitrippen Nuten vorzusehen, die in der inneren Seitenwand vorgesehen sind. Auch diese Maßnahme reduziert die potentielle Kontaktfläche zwischen dem Aromabehälter und einer möglichen Gestaltung der Trinkvorrichtung und, damit verbunden, die Reibung bei einer Relativbewegung zwischen beiden Bauteilen.

Wenn sich die Nuten in der inneren Seitenfläche des Aromabehälters ausreichend tief nach innen in Richtung auf die Aromakammer erstrecken, führt dies dazu, dass sich auf der der Aromakammer zugewandten Seite der inneren Seitenwand Erhebungen befinden, die sich in die Aromakammer erstrecken und ebenfalls durch die wiederholte Ablösung der Strömung des Luftstroms entlang der inneren Seitenwand die Durchmischung des den Aromabehälter durchströmenden Luftstroms unterstützten.

In gleicher Weise ist es auch möglich, die Reibung zu reduzieren, indem bereichsweise die innere Seitenwand des Aromabehälters nicht der äußeren Formgebung des Kopfteils der Trinkvorrichtung entspricht und daher keine vollflächige Anlage zwischen dem Aromabehälter und einer Trinkvorrichtung besteht.

Vorzugsweise befindet sich die Lufteintrittsöffnung im Bereich der unteren Wand des Aromabehälters, und die Luftaustrittsöffnung ist in einer Seitenwand des Aromabehälters angeordnet, vorzugsweise in einer inneren Seitenwand im Falle einer im Wesentlichen ringförmigen Geometrie des Aromabehälters mit einer äußeren Seitenwand und einer inneren Seitenwand.

Das Vorsehen der Lufteintrittsöffnung im Bereich der unteren Wand des Aromabehälters ist vorteilhaft, wenn der Aromabehälter auf das Kopfteil einer Trinkvorrichtung aufgesteckt werden soll und dort in dichtenden Kontakt mit dem Kopfteil der Trinkvorrichtung treten soll. Darüber hinaus kann durch das Vorsehen der Lufteintrittsöffnung im Bereich der unteren Wand des Aromabehälters und je nach Anordnung der Trägersubstanz im Aromabehälter eine Durchströmung des Strömungsbehälters erzeugt werden, bei welcher der Luftstrom beim Durchströmen des Aromabehälters die Trägersubstanz durchströmen muss und nicht unmittelbar in den im Aromabehälter vorgesehenen Kopfraum strömt.

Das Vorsehen der Luftaustrittsöffnung in einer Seitenwand des Aromabehälters eröffnet die Möglichkeit, eine definierte und lange Wegstrecke des den Aromabehälter durchströmenden Luftstroms zu generieren. Beispielsweise kann sich die Luftaustrittsöffnung in einem Bereich befinden, in dem die Trägersubstanz in der Aromakammer an der Luftaustrittsöffnung anliegt. Auf diese Weise muss der den Aromabehälter durchströmende Luftstrom vor dem Austritt aus dem Aromabehälter die Trägersubstanz durchströmen und kann von dort aus durch die Luftaustrittsöffnung austreten.

Im Falle einer im Wesentlichen ringförmigen Geometrie des Aromabehälters mit einer äußeren Seitenwand und einer inneren Seitenwand befindet sich die Luftaustrittsöffnung vorzugsweise in der inneren Seitenwand des Aromabehälters, so dass die aus dem Aromabehälter austretende, aromatisierte Luft in einen Transportkanal für Trinkflüssigkeit eintreten kann, der sich durch den von der inneren Seitenwand des Aromabehälters umschlossenen Raum erstreckt.

Nach einer bevorzugten Ausführungsform der Erfindung umfasst der Aromabehälter eine Unterschale und eine mit der Unterschale verbundene Oberschale, wobei die Luftaustrittsöffnung im Verbindungsbereich zwischen Oberschale und Unterschale angeordnet ist.

Diese Gestaltung hat den Vorteil, dass sie technisch einfach im Rahmen der Herstellung mit einem Spritzgussverfahren umsetzbar ist. Es ist für das Herstellen der Luftaustrittsöffnung kein Schieber erforderlich.

Vorzugsweise befindet sich im Kontaktbereich zwischen Unterschale und Oberschale eine umlaufende Nut, die als Schattenfuge dient und mehrere Funktionen erfüllen kann. Zum einen kann eine derartige Schattenfuge dem Benutzer die Position eines relativ zum Kopfteil einer Trinkvorrichtung verschiebbaren Aromabehälters anzeigen. Zum anderen lassen sich die Unterschale und Oberschale durch Ultraschallschweißen miteinander verbinden, ohne dass die Gefahr besteht, dass sich eine mögliche Materialanhäufung im Bereich der Schweißnaht über die Außenfläche der äußeren Seitenwand nach außen erstreckt.

Nach einem zweiten Aspekt der Erfindung betrifft diese eine Trinkvorrichtung, umfassend einen erfindungsgemäßen Aromabehälter, sowie ein Kopfteil, mit dem der Aromabehälter so verbindbar ist, dass zumindest ein Teil des Aromabehälters von einer aktivierten Position in eine nicht aktivierte Position bewegbar ist, wobei in der aktivierten Position die Luftaustrittsöffnung in Strömungsverbindung mit einem Transportkanal für Trinkflüssigkeit in der Trinkvorrichtung steht, und in der nicht aktivierten Position keine Strömungsverbindung zwischen der Luftaustrittsöffnung und dem Transportkanal für Trinkflüssigkeit besteht, und die Lufteintrittsöffnung im Wesentlichen abgedichtet ist.

Dabei ist es bei der Bewegung von einer aktivierten Position in eine nicht aktivierte Position möglich, dass der Aromabehälter vollständig bewegt wird, z.B. durch eine Translationsbewegung oder durch eine Rotationsbewegung. Ebenso ist es möglich, dass nur zumindest ein Teil des Aromabehälters von einer aktivierten Position in eine nicht aktivierte Position bewegbar ist. So können beispielsweise zwei gegeneinander bewegbare Teile des Aromabehälters relativ zueinander bewegt werden, indem diese relativ zueinander gedreht werden. Ebenso ist es auch möglich, zwei Teile des Aromabehälters durch ein Auseinanderziehen oder ein Zusammendrücken zu aktivieren, wobei durch das Auseinanderziehen der beiden Teile die Luftaustrittsöffnung und/oder die Lufteintrittsöffnung in den jeweiligen Teilen des Aromabehälters in eine miteinander fluchtende Position gebracht werden können. Im einfachsten Fall können bei einem ringförmigen Aromabehälter zwei Gehäuseteile gegeneinander verdrehbar sein und nur in einer definierten Verdrehposition sind Luftaustrittsöffnungen, die in beiden Gehäuseteilen vorgesehen sind, in einer miteinander fluchtenden Position und der Aromabehälter in der aktivierten Position.

Die Trinkvorrichtung ist weiterhin dadurch gekennzeichnet, dass in der aktivierten Position die Luftaustrittsöffnung des Aromabehälters in Strömungsverbindung mit einem Transportkanal für Trinkflüssigkeit steht. Der aromatisierte Luftstrom wird demnach nicht an die umgebende Luft abgegeben, um bei einem Benutzer orthonasal zu wirken, sondern wird in den Transportkanal für Trinkflüssigkeit eingespeist, so dass die aromatisierte Luft retronasal vom Benutzer wahrgenommen wird und der Sinneseindruck einer Geschmackswahrnehmung der Trinkflüssigkeit entsteht.

Vorzugsweise umfasst die Trinkvorrichtung einen abnehmbaren Deckel, der auf dem Kopfteil der Trinkvorrichtung anbringbar ist, wobei der abnehmbare Deckel mindestens ein kraftausübendes Element umfasst, mit dem der Aromabehälter beim Abnehmen des Deckels von einer nicht aktivierten Position in die aktivierte Position bewegbar ist.

Das Bewegen des Aromabehälters zwischen der nicht aktivierten Position und der aktivierten Position verlängert die Gebrauchsdauer des Aromabehälters, da dieser nicht unbeabsichtigt ausdünstet, wenn er nicht im Betrieb ist. Die Bewegung des Aromabehälters zwischen der nicht aktivierten Position und der aktivierten Position kann der Benutzer selbst vornehmen, jedoch besteht stets die Gefahr, dass der Benutzer dies vergisst. Daher ist die Lösung von Vorteil, die Trinkvorrichtung mit einem abnehmbaren Deckel zu versehen. Dieser muss abgenommen werden, wenn aus der Trinkvorrichtung getrunken werden soll. Wenn die Trinkvorrichtung ein kraftausübendes Element umfasst, mit dem der Aromabehälter beim Abnehmen des Deckels von der nicht aktivierten Position selbsttätig in die aktivierte Position bewegbar ist, ist nach dem Abnehmen des Deckels die Trinkvorrichtung automatisch in einem gebrauchsfertigen Zustand mit dem Aromabehälter in der aktivierten Position.

In gleicher Weise ist es auch möglich, das Aufsetzen des Deckels nach dem Trinkvorgang dazu zu verwenden, um den Aromabehälter in die nicht aktivierte Position zu bringen.

Nach einer bevorzugten Ausführungsform der Erfindung umfasst das mindestens eine kraftausübende Element eine Anlagefläche am Deckel, mit welcher der Aromabehälter bei einer Rotation des Deckels beim Abschrauben des Deckels von der nicht aktivierten Position in die aktivierte Position drehbar ist. Mit anderen Worten wird die Rotationsbewegung des Deckels beim Abschrauben dazu verwendet, den Aromabehälter von der nicht aktivierten Position in die aktivierte Position zu drehen. Dieses Wirkprinzip kann in gleicher Weise dazu verwendet werden, um beim Aufschrauben des Deckels ebenfalls mit Hilfe einer Anlagefläche am Deckel den Aromabehälter von der aktivierten Position in die nicht aktivierte Position zu drehen.

Nach einer alternativen Ausgestaltung der Erfindung umfasst die Seitenwand des Aromabehälters zumindest abschnittsweise einen Vorsprung, der sich nach außen erstreckt. Das mindestens eine kraftausübende Element umfasst ein Eingriffselement am Deckel, vorzugsweise ein hakenförmiges Element, das angeordnet und gestaltet ist, um den Vorsprung während des Abnehmens des Deckels formschlüssig zu umgreifen und den Aromabehälter von der nicht aktivierten Position in die aktivierte Position zu bewegen.

Dazu kann ein einzelnes, umlaufendes hakenförmiges Element oder es können mehrere hakenförmige Elemente am Deckel vorgesehen sein, die beim Aufschrauben des Deckels auf die Trinkvorrichtung auf der Unterseite des Vorsprungs an der Seitenwand des Aromabehälters einschnappen und beim Abnehmen des Deckels entweder durch Aufziehen oder Aufschrauben den Aromabehälter mit sich ziehen und von der nicht aktivierten Position in die aktivierte Position bringen. Am Ende des Aufschraubvorgangs kann dabei in der aktivierten Position der Aromabehälter gegen ein Anschlagelement anstoßen, so dass im letzten Abschnitt des Aufdrehens des Deckels die hakenförmigen Elemente elastisch verformen und wieder den Vorsprung an der Seitenwand des Aromabehälters freigeben, so dass der Deckel entfernt werden kann.

Nach einer alternativen Ausführungsform der Erfindung umfasst das kraftausübende Element ein elastisches Vorspannelement, das zwischen dem Aromabehälter und dem Kopfteil der Trinkvorrichtung angeordnet ist und den Aromabehälter bei abgenommenem Deckel in die aktivierte Position vorspannt.

Es kann sich bei dem elastischen Vorspannelement dabei um ein Teil handeln, das einstückig mit dem Kopfteil der Trinkvorrichtung ausgebildet ist, oder aber als separates Element vorgesehen ist. Auch bei dieser Lösung wird beim Abnehmen des Deckels der Aromabehälter ohne weiteres Zutun des Benutzers von der nicht aktivierten Position in die aktivierte Position bewegt.

Kurze Beschreibung der Zeichnungen

Nachfolgend wird die Erfindung anhand einiger Ausführungsbeispiele beschrieben. Dabei zeigen:

Fig. 1            eine Gesamtansicht eines Aromabehälters nach einer ersten Ausführungsform der Erfindung;

Fig. 2            eine Draufsicht auf den Aromabehälter nach Fig. 1;

Fig. 3      eine Schnittdarstellung des Aromabehälters entlang der Schnittlinie A-A in Fig. 2;

Fig. 4      eine Schnittdarstellung des Aromabehälters entlang der Schnittlinie B-B in Fig. 2;

Fig. 5      eine Gesamtansicht einer zweiten Ausführungsform eines erfindungsgemäßen Aromabehälters;

Fig. 6      eine Schnittdarstellung des Aromabehälters nach Fig. 5 analog der anhand der ersten Ausführungsform in Fig. 2 dargestellten Schnittebene A-A;

Fig. 7      eine Schnittdarstellung des Aromabehälters nach Fig. 5 analog der anhand der ersten Ausführungsform in Fig. 2 dargestellten Schnittebene B-B;

Fig. 8      eine Ansicht von unten des Aromabehälters nach Fig. 5;

Fig. 9 und Fig. 10      eine Draufsicht sowie eine dreidimensionale Darstellung eines Aromabehälters nach einer weiteren Ausführungsform der Erfindung;

Fig. 11 und Fig. 12      eine Draufsicht sowie eine dreidimensionale Darstellung eines Aromabehälters nach einer weiteren Ausführungsform der Erfindung;

Fig. 13 und Fig. 13a      eine dreidimensionale Ansicht sowie eine Draufsicht auf einen Aromabehälter nach einer weiteren Ausführungsform der Erfindung in der aktivierten Position;

Fig. 14 und Fig. 14a      eine dreidimensionale Ansicht sowie eine Draufsicht auf den Aromabehälter nach Fig. 13a in der nicht aktivierten Position;

Fig. 15 und Fig. 16      eine Draufsicht sowie eine dreidimensionale Ansicht eines Aromabehälters nach einer weiteren alternativen Ausführungsform der Erfindung;

Fig. 17 und Fig. 18      eine weitere Ausführungsform eines erfindungsgemäßen Aromabehälters in einer ersten, nicht aktivierten Position;

Fig. 19      den Aromabehälter nach Fig. 17 in einer aktivierten Position;

Fig. 20      eine Detaildarstellung im Schnitt des Aromabehälters nach Fig. 19 in der aktivierten Position;

Fig. 21 und Fig.22      eine Ausführungsform der erfindungsgemäßen Trinkvorrichtung mit einem Deckel zur automatischen Betätigung des Aromabehälters;

Fig. 23      eine Ausführungsform der erfindungsgemäßen Trinkvorrichtung mit einer ersten alternativen Möglichkeit eines kraftausübenden Elements;

Fig. 24      eine Ausführungsform der erfindungsgemäßen Trinkvorrichtung mit einer zweiten alternativen Möglichkeit eines kraftausübenden Elements;

Fig. 25 und Fig. 26      eine Draufsicht und Seitenansicht des kraftausübenden Elements nach Fig. 24;

Fig. 27      die Darstellung eines Deckels einer erfindungsgemäßen Trinkvorrichtung zur automatischen Betätigung des Aromabehälters;

Fig. 28      eine Ansicht von unten des Deckels nach Fig. 247

Fig. 29      eine Schnittansicht in der Richtung A-A in Fig. 28; und

Fig. 30      eine Schnittdarstellung, die das Zusammenwirken des Deckels nach Fig. 27 mit einem Aromabehälter nach Fig. 5 erläutert.

Wege zur Ausführung der Erfindung

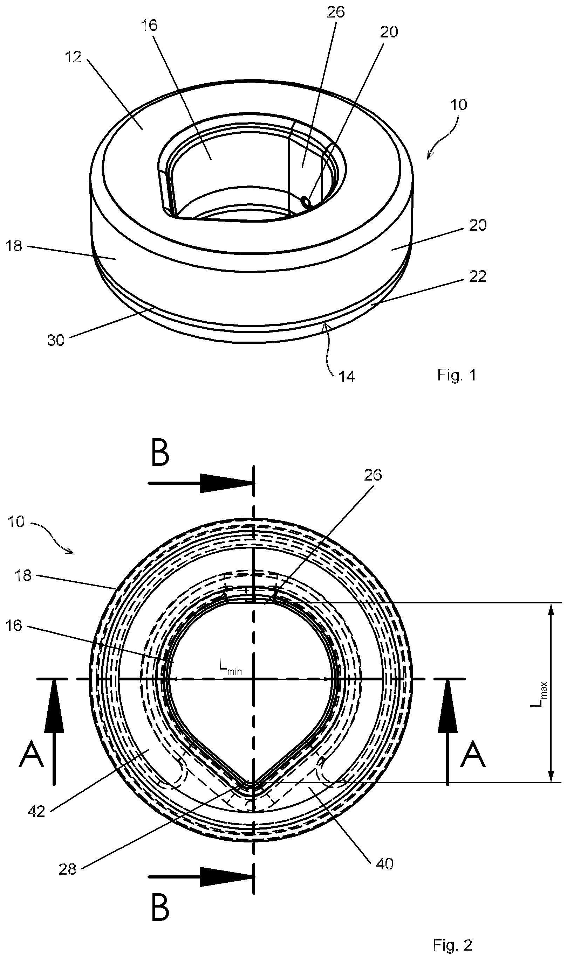

Nachfolgend wird die Erfindung rein beispielhaft anhand der in den Figuren dargestellten Ausführungsformen erläutert. Dabei werden Begriffe wie oben, unten und seitlich so verwendet, als würde der Aromabehälter beispielsweise nach Fig. 1 mit seiner unteren Wand auf einer horizontalen, ebenen Oberfläche liegen.

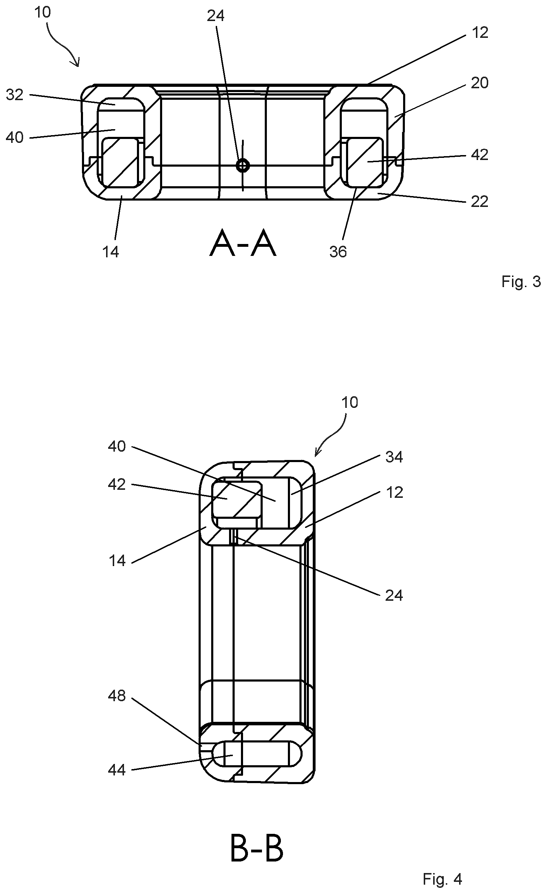

Ein Aromabehälter 10 nach einer ersten Ausführungsform ist in den Fig. 1 bis 4 dargestellt. Der Aromabehälter 10 weist dabei eine obere Wand 12, eine untere Wand 14, eine innere Seitenwand 16 sowie eine äußere Seitenwand 18 auf. Wie insbesondere in den Schnittdarstellungen nach Fig. 3 und 4 gezeigt ist, umschließen die obere Wand 12, untere Wand 14, innere Seitenwand 16 sowie äußere Seitenwand 18 eine Aromakammer 40.

Die obere Wand 12 ist vorzugsweise zumindest bereichsweise eben, damit ein Etikett an der Außenseite der oberen Wand 12 anbringbar ist.

Der Aromabehälter 10 nach Fig. 1 ist im Wesentlichen ringförmig mit einer kreisringförmigen äußeren Seitenwand 18 und einer inneren Seitenwand 16, die von einer Kreisform abweicht. Mit anderen Worten besitzt der von der inneren Seitenwand 16 umschlossene Raum eine Querschnittsfläche, die nicht kreisförmig ist.

Im konkreten Beispiel ist, wie am besten in der Darstellung nach Fig. 2 ersichtlich ist, die innere Seitenwand 16 mit einer Abflachung 26 versehen und weist ansonsten angenähert eine Tropfenform auf mit einem im Wesentlichen spitz zulaufenden Ende 28. Wie aus Fig. 2 ersichtlich ist, erlaubt die dargestellte Geometrie der inneren Seitenwand 16 eine eindeutige Positionierung des Aromabehälters an einer nicht dargestellten Trinkvorrichtung. Dabei kann der Aromabehälter 10 auf ein entsprechend geformtes Element der Trinkvorrichtung aufgesetzt werden, dessen Außenkontur der Geometrie der inneren Seitenwand soweit folgt, dass der Aromabehälter nur in einer einzigen Winkelposition, d.h. in Bezug auf eine Drehung in der Zeichenebene der Fig. 2, auf der Trinkvorrichtung aufgesetzt werden kann.

Die Geometrie der inneren Seitenwand 16 ist dabei so gewählt, dass die Abweichung der Innenkontur von einer Kreisringform nur gering ist. Das Verhältnis zwischen der größten lichten Abmessung $L_{max}$ in der Querschnittsfläche des durch die inneren Seitenwand 16 umschlossenen Raumes im Verhältnis zu der geringsten Abmessung $L_{min}$ bewegt sich dabei im Bereich zwischen 1.05 und 1.15 und beträgt bevorzugt etwa 1.1.

Die Abflachung 26 dient dazu, die Luftaustrittsöffnung 24 beim Aufschieben des Aromabehälters auf eine entsprechend gestaltete Geometrie der Trinkvorrichtung abdichten zu können. Dafür eignet sich das Vorsehen einer ebenen Teilfläche 26 im Bereich der Luftaustrittsöffnung 24 besser als eine gerundete Fläche.

Die Luftaustrittsöffnung 24 ist im Nahtbereich zwischen einer oberen Schale 20 und einer unteren Schale 22 vorgesehen. Dies besitzt den Vorteil, dass der Aromabehälter leicht gefertigt werden kann, da kein separater Schieber bei einer Spritzgussfertigung vorgesehen sein muss, sondern die obere Schale 20 und untere Schale 22 gemeinsam die Luftaustrittsöffnung 24 bilden.

Im Bereich zwischen der oberen Schale 20 und unteren Schale 22 ist eine Schattenfuge 30 vorgesehen, die verschiedene Aufgaben erfüllt. Zum einen kann das Vorsehen einer Schattenfuge zwischen oberer Schale 20 und unterer Schale 22 verhindern, dass bei dem Verschweißen der oberen Schale mit der unteren Schale austretendes Material das optische Erscheinungsbild des Aromabehälters beeinträchtigt. Darüber hinaus lassen sich durch das Vorsehen der Schattenfuge abgerundete Kanten realisieren. Ein weiterer Vorteil des Vorsehens der Schattenfuge 30 besteht darin, dass dem Benutzer visuell ein Feedback darüber gegeben werden kann, in welcher Betriebsposition sich der Aromabehälter befindet, indem dieser in einer vertikalen Richtung relativ zur Trinkvorrichtung verschoben werden kann und dabei in einer vertikal nach unten verschobenen Anordnung des Aromabehälters die Schattenfuge für den Benutzer nicht mehr sichtbar ist.

Die Kontur der inneren Seitenwand 16 ist vertikal, **d.h.** der von der inneren Seitenwand umschlossene Raum hat eine über der Höhe des Aromabehälters konstante Querschnittsabmessung. Dies ermöglicht ein vertikales Verschieben des Aromabehälters relativ zu einem Element einer Trinkvorrichtung, das sich durch den von der inneren Seitenwand umschlossenen Raum erstreckt und an der inneren Seitenwand anliegt. Das Vorsehen einer inneren Seitenwand mit vertikaler Erstreckung ist bei allen Ausführungsformen der Erfindung vorteilhaft.

Die Lufteintrittsöffnung 48 ist in der Fig. 4 dargestellt und befindet sich in der unteren Wand 14 des Aromabehälters. Bevorzugt befindet sich die Lufteintrittsöffnung 48 dabei in der unteren Wand 14 im Bereich des spitz zulaufenden Endes 28 und damit diametral entgegengesetzt zu der Luftaustrittsöffnung 24. Als Folge daraus muss der den Aromabehälter durchströmende Luftstrom eine möglichst weite Wegstrecke zurücklegen und kann auf diese Weise auf eine besonders wirksame Weise mit Aromasubstanz angereichert werden.

Wie in den Schnittdarstellungen nach Fig. 3 und 4 dargestellt ist, befindet sich im Inneren des Aromabehälters 10 die Aromakammer 40. Zudem ist in der Aromakammer 40 eine Trägersubstanz 42 eingesetzt, welche die Aromasubstanz trägt. Die Trägersubstanz ist, wie in Fig. 2 dargestellt ist, U-förmig, d.h. nicht vollständig umlaufend, wodurch sich beim Produktionsprozess das maschinelle Einlegen der Trägersubstanz in die untere Schale 22 des Aromabehälters vor dem Aufsetzen der oberen Schale 20 erleichtern lässt. Die U-förmige Geometrie der Trägersubstanz ermöglicht darüber hinaus eine bessere Aromaentfaltung, da im Ausführungsbeispiel nach Fig. 1 bis 4 im Bereich des spitz zulaufenden Endes 28 die Luft zunächst in einen luftgefüllten Raum 44 der Aromakammer 40 eintritt und anschließend gleichmäßig über die gesamte Höhe der Trägersubstanz 42 in diese eintreten und die Trägersubstanz durchströmen kann.

Wie ebenfalls in den Fig. 3 und 4 dargestellt ist, befindet sich oberhalb der Trägersubstanz ein Kopfraum, in dem die Aromakammer 40 nicht mit Trägersubstanz 42 gefüllt ist. Auch das Vorsehen eines Kopfraums besitzt die Funktion, ein besseres Anreichern der Luft sowie eine Homogenisierung der Luft mit Aromasubstanz zu ermöglichen.

Wie in Fig. 3 und 4 dargestellt ist, ist die Trägersubstanz 42 dabei so in die Aromakammer 40 eingesetzt, dass die Trägersubstanz auf der der Aromakammer zugewandten Seite 36 der unteren Wand 14 aufliegt. Der Kopfraum 32 ist folglich zwischen der Trägersubstanz 42 und der der Aromakammer zugewandten Seite 34 der oberen Wand 12 vorgesehen. Der Kopfraum 32 besitzt dabei eine Höhe, die geringer ist als 50% der Höhe der Aromakammer 40, d.h. weniger als 50% des Abstands zwischen der der Aromakammer zugewandten Seite 34 der oberen Wand 12 sowie der der Aromakammer zugewandten Seite 36 der unteren Wand 14. Es ist allerdings bevorzugt, dass die Trägersubstanz mindestens 80% der Höhe der Aromakammer einnimmt.

Die Trägersubstanz ist in allen dargestellten Ausführungsformen ein Vliesmaterial, das bei 100Pa Differenzdruck eine Luftdurchlässigkeit L von $L \geq 200$ $1/(m^2 \cdot s)$ und bevorzugt zwischen 220 $1/(m^2 \cdot s)$ und 280 $1/(m^2 \cdot s)$ besitzt. Vorzugsweise besteht das Vlies aus 100% Polyester. Die Porosität des Vliesmaterials liegt dabei zwischen 70% und 93% und bevorzugt zwischen 70% und 80%.

Der Durchmesser der Lufteintrittsöffnung in der unteren Wand liegt im Ausführungsbeispiel nach den Fig. 1 bis 4 bei 1.2mm.

Indem die obere Schale 20 und untere Schale 22 per Ultraschallschweißverfahren miteinander verbunden werden, werden die obere Schale und untere Schale außer im Bereich der Luftaustrittsöffnung umlaufend dicht verbunden, so dass ein potentielles Eindringen von Luft in den Aromabehälter oder ein ungewünschtes Austreten von Aromasubstanz, beispielsweise während der Lagerung, aus dem Aromabehälter vermieden werden kann.

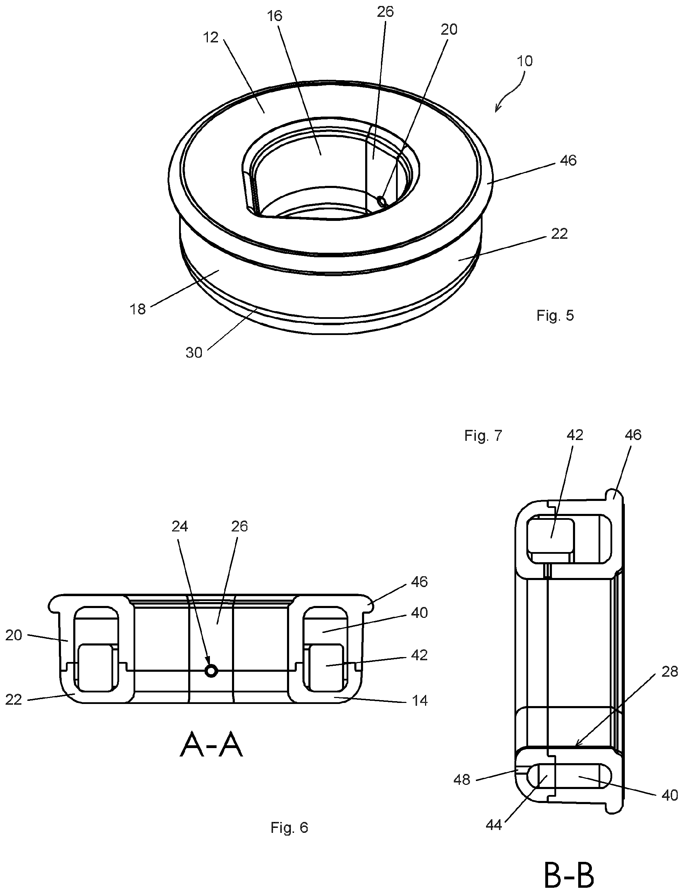

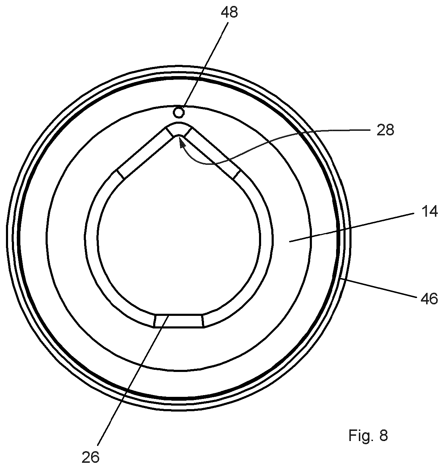

Die in den Fig. 5 bis 8 dargestellte Ausführungsform des Aromabehälters 10 unterscheidet sich von dem Aromabehälter nach den Fig. 1 bis 4 lediglich dadurch, dass an der äußeren Seitenwand 18 des Aromabehälters ein vorstehender Bereich 46 vorgesehen ist. Beim Ausführungsbeispiel der Fig. 5 und 8 ist der vorstehende Bereich 46 als umlaufender Rand ausgestaltet, kann in gleicher Weise aber auch einzelne, getrennte Teilabschnitte umfassen. In gleicher Weise ist im Ausführungsbeispiel nach den Fig. 5 und 8 der umlaufende Rand in eine Ebene mit der oberen Wand 12 angeordnet, was ebenfalls nur ein Beispiel darstellt. Es ist in gleicher Weise möglich, den oder die vorstehenden Abschnitte an einer beliebigen Position der Seitenwand vorzusehen. Schließlich sollte auch deutlich sein, dass die Gesamtgeometrie des in den Fig. 5 bis 8 dargestellten Aromabehälters ebenfalls nur beispielhaft zu verstehen ist. Der Aromabehälter muss keine Ringform besitzen. Wichtig ist ausschließlich, dass die Seitenwand zumindest abschnittsweise einen Vorsprung umfasst. Dabei ist es bevorzugt, dass sich der Vorsprung im Wesentlichen senkrecht zur äußeren Seitenwand nach außen erstreckt.

Der bei der Ausführungsform nach Fig. 5 bis 8 dargestellte umlaufende Rand 46 dient dazu, dass der Aromabehälter von einem Benutzer leichter gegriffen werden kann. In einer speziellen, später erläuterten Ausgestaltung im Zusammenwirken mit einer Trinkvorrichtung kann der umlaufende Rand 46 auch dazu verwendet werden, den Aromabehälter in einer bequemen Weise zwischen einer nicht aktivierten Position und einer aktivierten Position zu bewegen oder von der Trinkvorrichtung zu trennen.

Der umlaufende Rand 46 muss nur eine geringe radiale Erstreckung besitzen, um einem Benutzer das Greifen des Aromabehälters zu erleichtern. So genügt in der Regel eine Erstreckung von 0.6 mm bis 1.5 mm nach außen bereits, um dem Benutzer zusätzlichen Halt beim Greifen und nach oben Abziehen des Aromabehälters zu bieten. Auch in Bezug auf die vertikale Höhe des umlaufenden Rands ist es ausreichend, wenn der umlaufend Rand eine Dicke, d.h. vertikale Erstreckung zwischen 2.0 mm und 2.5 mm besitzt. Bei gängigen Kunststoffmaterialien, aus denen der Aromabehälter vorzugsweise hergestellt ist, lässt sich durch das Vorsehen einer Dicke des umlaufenden Rands in diesem Bereich eine ausreichende Stabilität erzielen.

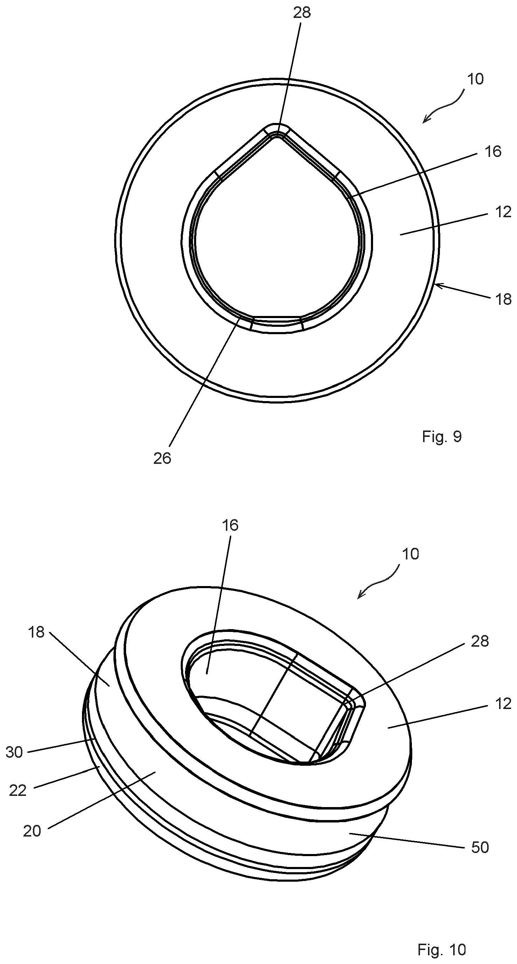

In den Fig. 9 und 10 ist eine Draufsicht sowie eine dreidimensionale Ansicht einer weiteren alternativen Ausgestaltung eines erfindungsgemäßen Aromabehälters gezeigt. Der Aromabehälter nach den Fig. 9 und 10 ist ähnlich aufgebaut wie der Aromabehälter nach den vorangehenden Ausführungsformen, besitzt jedoch eine Griffmulde 50 im Bereich der Seitenwand 18. Auch der Aromabehälter nach den Fig. 9 und 10 kann dabei mit einem abschnittsweise vorgesehenen oder umlaufenden Rand wie bei der Ausführungsform nach Fig. 5 bis 8 versehen sein. Die Griffmulde 50 dient dazu, dass der Benutzer beim Aufschieben des Aromabehälters auf das Kopfteil einer zugehörigen Trinkvorrichtung den Aromabehälter besser greifen kann, wodurch sich die Betätigung des Aromabehälters vereinfacht.

Neben einem vorstehenden Abschnitt wie der beispielhaft dargestellte umlaufende Rand 46 sowie einer Griffmulde 50 sind auch andere Geometrien denkbar, die für ein leichteres Greifen des Aromabehälters sorgen. Beispielsweise kann dies auch durch das Vorsehen einer geeigneten Oberflächenstruktur der Seitenwand wie z.B. einer texturierten Fläche, oder der Verwendung von Beschichtungen, beispielsweise mit Elastomeren, erfolgen.

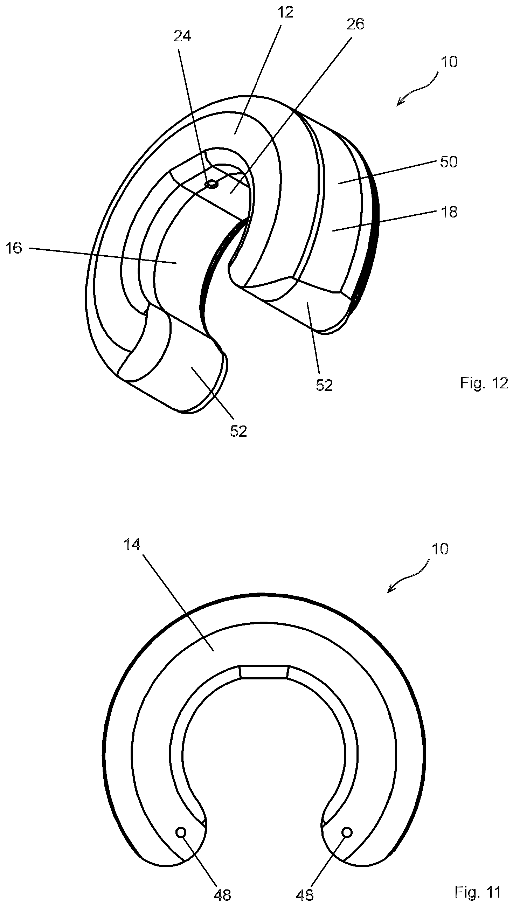

In zahlreichen Ausführungsformen wird jeweils auf eine im Wesentlichen kreisringförmige Geometrie des Aromabehälters mit einer Kreisringform außen an einer angenäherten Tropfenform innen Bezug genommen, um besser auf die spezifischen Unterschiede zwischen den einzelnen Ausführungsformen hinweisen zu können. Dennoch sollte ersichtlich sein, dass der erfindungsgemäße Aromabehälter nicht auf eine derartige Geometrie beschränkt ist. Als Beispiel für eine unterschiedliche Geometrie wird im Folgenden auf die Fig. 11 und 12 verwiesen, die einen Aromabehälter 10 darstellen, der im Wesentlichen U-förmig gestaltet ist.

Der Aromabehälter nach den Fig. 11 und 12 weist wieder eine obere Wand 12, eine untere Wand 14, eine innere Seitenwand 16 sowie eine äußere Seitenwand 18 auf. Die äußere Seitenwand 18 kann wie bei der Ausführungsform des Aromabehälters nach den Fig. 9 und 10 mit einer konkav in der Seitenwand 18 angeordneten Griffmulde 50 versehen sein. In gleicher Weise könnte auch der Aromabehälter nach den Fig. 11 und 12 mit einem umlaufenden Rand 46 versehen sein, wie anhand der Ausführungsformen nach Fig. 5 bis 8 beschrieben wurde. Die Luftaustrittsöffnung 24 ist, wie in Fig. 12 dargestellt ist, in der Mitte der U-förmigen Geometrie des Aromabehälters im Bereich einer Abflachung 26 vorgesehen.

Abweichend zu den vorangegangenen Ausführungsformen ist der Aromabehälter 10 nach Fig. 11 und 12 mit zwei Lufteintrittsöffnungen 48 versehen, die jeweils im Bereich der freien Enden 52 des U-förmigen Profils liegen und sicherstellen, dass die in der Trägersubstanz der Aromakammer befindliche Aromasubstanz vom Bereich beider freien Enden 52 her von dem durchströmenden Luftstrom aufgenommen wird.

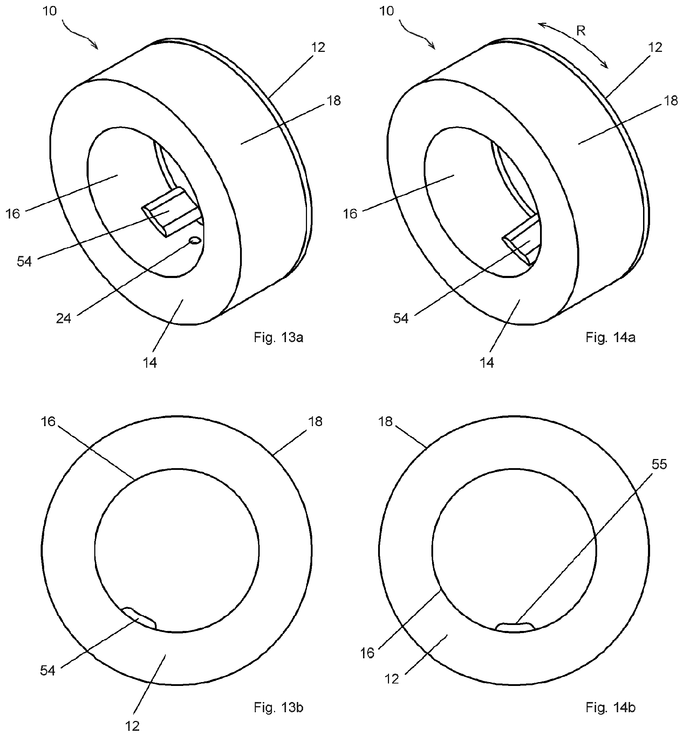

Der in den Fig. 13a, 13b, 14a und 14b dargestellte Aromabehälter 10 weist einen Verschlussansatz 54 auf, der sich im Bereich der inneren Seitenwand 16 im Bereich der Luftaustrittsöffnung 24 befindet. Der Verschlussansatz 54 im dargestellten Ausführungsbeispiel ist fest mit der oberen Wand 12 verbunden, jedoch kann in gleicher Weise das nachfolgend beschriebene Wirkprinzip auch verwirklicht werden, wenn der Verschlussansatz anstatt mit der oberen Wand fest mit der unteren Wand verbunden ist, Der Verschlussansatz erstreckt sich entlang der inneren Seitenwand 16 in den durch die innere Seitenwand umschlossenen Raum hinein und ist mit engem Abstand entlang der inneren Seitenwand angeordnet.

Der Verschlussansatz 54 dient dazu, den Aromabehälter 10 zwischen einer in den Fig. 13a und 13b dargestellten aktivierten Position und einer in den Figuren 14a und 14b dargestellten, nicht aktivierten Position zu bewegen. In der aktivierten Position liegt die Luftaustrittsöffnung 24 frei, während in der nicht aktivierten Position die Luftaustrittsöffnung 24 durch den Verschlussansatz 54 verschlossen ist.

Dazu ist die obere Wand 12 relativ zu demjenigen Wandabschnitt der inneren Seitenwand 15, in dem sich die Luftaustrittsöffnung 24 befindet, rotierbar. Der Benutzer kann somit durch Rotation der oberen Wand 12 in Rotationsrichtung R den Aromabehälter 10 zwischen der in Fig. 13a dargestellten aktivierten Position und der in Fig. 14a dargestellten nicht aktivierten Position bewegen.

Die Rotation R kann dabei entweder bewirkt werden, indem ein Benutzer den Aromabehälter an der oberen Wand 12 greift und diese relativ zur unteren Wand 14 verdreht. Alternativ könnte auch der Benutzer mit einem Finger den Verschlussansatz 54 verschieben. Dazu kann der Verschlussansatz, wie im dargestellten Ausführungsbeispiel, eine konkav geformte Grifffläche 55 aufweisen und in einer anderen, geeigneten Weise den Kontakt zwischen einem Finger und dem Verschlussansatz 54 verbessern.

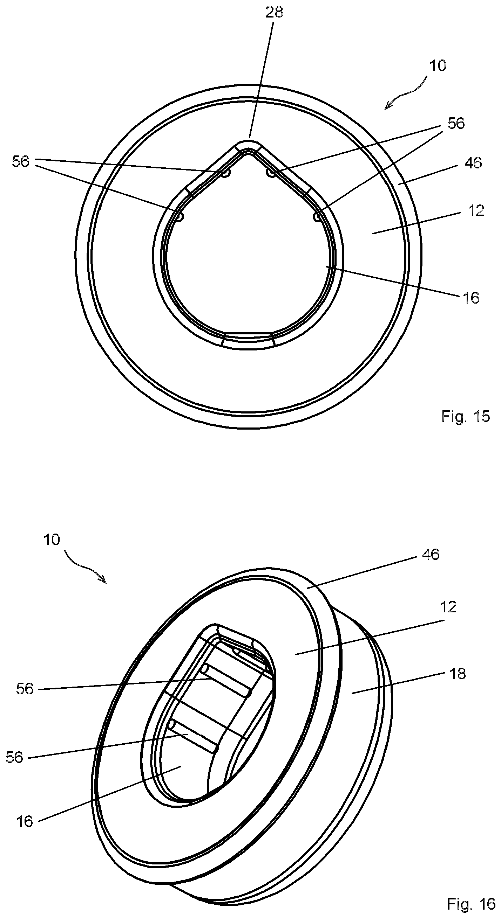

In den Fig. 15 und 16 ist eine Ausführungsform des erfindungsgemäßen Aromabehälters dargestellt, bei dem sich von der inneren Seitenwand 16 Gleitrippen 56 in den von der inneren Seitenwand umschlossenen Raum erstrecken. Die Gleitrippen erstrecken sich dabei in vertikaler Richtung und besitzen eine abgerundete, von der inneren Seitenwand 16 weg weisende Kontur auf, so dass im Bereich der Gleitrippen 56 im Wesentlichen nur eine Linienberührung zwischen dem Aromabehälter 10 und dem Kopfteil einer Trinkvorrichtung hergestellt werden kann, die sich durch den von der inneren Seitenwand 16 des Aromabehälters umschlossenen Raum erstreckt.

Die Gleitrippen 56, die sich in vertikaler Richtung des Aromabehälters erstrecken, dienen zusätzlich dazu, den Aromabehälter zu führen und ein gerades Abziehen des Aromabehälters von einer Trinkvorrichtung zu ermöglichen. In den nachfolgenden Ausführungsformen werden Gestaltungsvarianten dargestellt, bei denen ein Aromabehälter zwischen einer aktivierten Position und einer nicht aktivierten Position bewegbar ist.

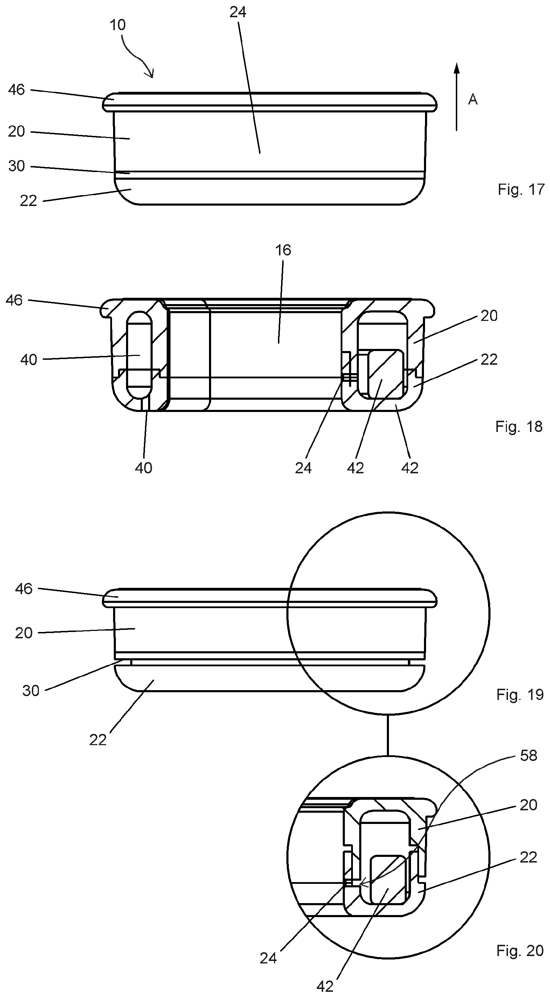

Bei der Ausführungsform nach den Fig. 17 bis 20 wird der Aromabehälter zwischen der aktivierten und nicht aktivierten Position bewegt, indem die obere Schale 20 und die untere Schale 22 des Aromabehälters 10 relativ zueinander bewegbar sind. Dazu sind, wie aus den Schnittdarstellungen in den Fig. 18 und 20 ersichtlich ist, die obere Schale 20 und die untere Schale 22 in vertikaler Richtung gegeneinander verschiebbar.

Der Aromabehälter kann dabei mit einer Trinkvorrichtung so zusammenwirken, dass die Trinkvorrichtung sich mit ihrem Kopfteil durch den von der inneren Seitenwand 16 umschlossenen Raum hindurchbewegt. Die untere Wand 14, in der sich die Lufteintrittsöffnung 48 befindet, kann dabei im nicht aktivierten Zustand, der in Fig. 18 dargestellt ist, auf dem Kopfteil der Trinkvorrichtung so aufliegen, dass die Lufteintrittsöffnung 48 abgedichtet ist. Darüber hinaus ist im nicht aktivierten Zustand auch die Luftaustrittsöffnung 24 verschlossen. Wird nun der Aromabehälter vertikal nach oben gezogen, indem entweder ein Benutzer oder aber, wie später erläutert werden wird, der Deckel der Trinkvorrichtung unter den umlaufenden Rand 46 greift und den Aromabehälter in Pfeilrichtung A nach oben zieht, werden die gegeneinander verschiebbar geführte obere Schale 20 und untere Schale 22 auseinandergezogen, wodurch sich der Durchgang 58 im Bereich der Luftaustrittsöffnung 24 bildet (siehe Fig. 20). Auf diese Weise werden bei der vertikalen Bewegung des Aromabehälters in Pfeilrichtung A sowohl die Lufteintrittsöffnung 48 als auch die Luftaustrittsöffnung 24 geöffnet und der Aromabehälter damit in eine betriebsbereite, aktivierte Position gebracht.

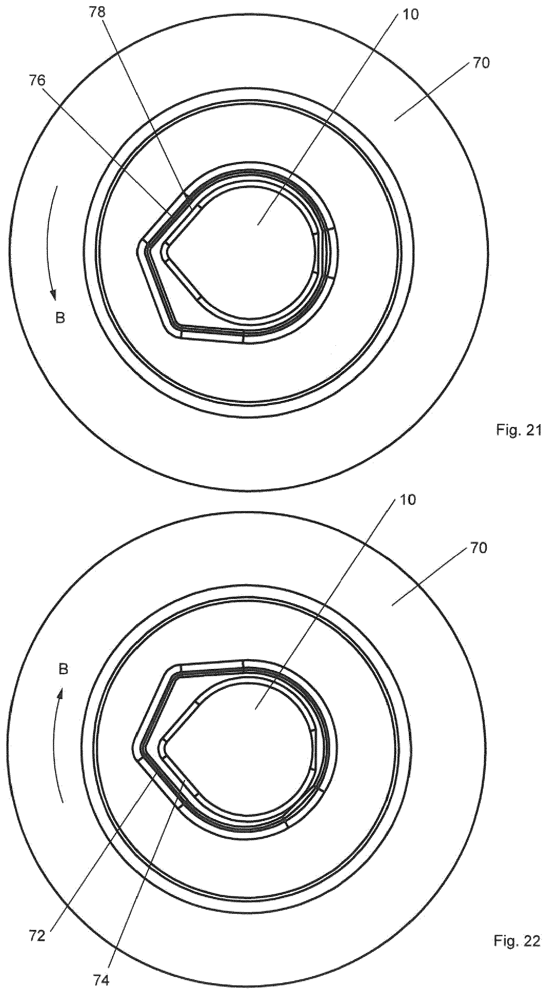

Die Ausführungsform nach Fig. 21 und 22 zeigt eine alternative Gestaltung eines Aromabehälters 10, der bei der Betätigung eines Deckels 70 der Trinkvorrichtung zwischen der aktivierten und der nicht aktivierten Position bewegbar ist. Dazu wird der Deckel 70 in Rotationsrichtung B entweder von der Trinkvorrichtung abgeschraubt oder auf diese aufgeschraubt. Dabei ist der Deckel so gestaltet, dass er frei und unabhängig von dem Aromabehälter bewegbar ist, jedoch im letzten Abschnitt des vollständigen Aufschraubens auf der Trinkvorrichtung mittels einer Mitnahmefläche 72 in Eingriff kommt mit einem oberen Randbereich 74 des Aromabehälters. Der Aromabehälter wird dabei beim Aufschrauben des Deckels auf die Trinkvorrichtung im Uhrzeigersinn soweit mitbewegt, dass sich der Aromabehälter in einem nicht aktivierten Zustand befindet, in dem die Luftaustrittsöffnung nicht mehr mit einer korrespondierenden Eintrittsöffnung in den Transportkanal für Trinkflüssigkeit fluchtet und daher keine aromatische Luft aus dem Aromabehälter abgegeben werden kann. Das Drehen des Aromabehälters kann in gleicher Weise auch manuell vom Benutzer durchgeführt werden.

In gleicher Weise kann bei dem Abschrauben des Deckels von der Trinkvorrichtung gemäß Fig. 21 die Mitnahmefläche 76 des Deckels mit dem oberen Randbereich 78 des Aromabehälters 10 in Eingriff kommen und den Aromabehälter entsprechend der Pfeilrichtung B in Fig. 21 soweit mitbewegen, bis sich der Aromabehälter in einer aktivierten Position befindet und der Deckel durch die Bewegung nach oben in der Zeichenebene der Fig. 21 mit seiner Mitnahmefläche 76 nicht länger in Eingriff ist mit dem oberen Randbereich 78, so dass der Aromabehälter somit während des weiteren Drehens des Deckels in seiner aktivierten Position verbleibt.

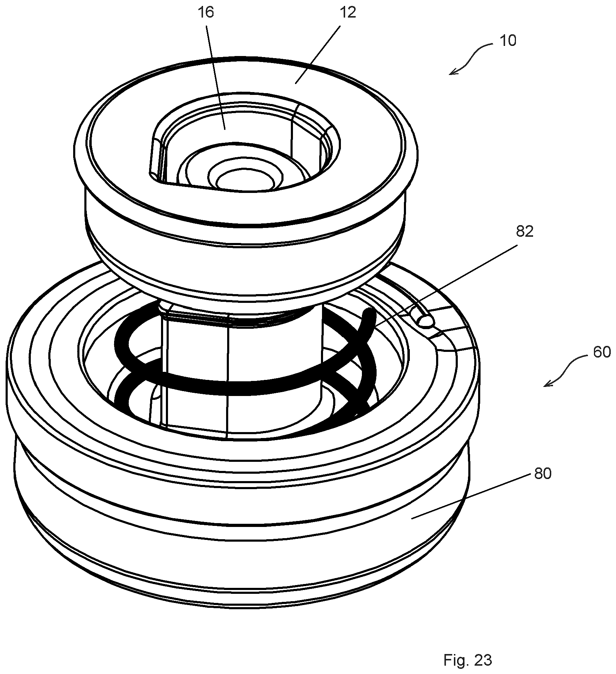

In der Fig. 23 ist eine alternative Möglichkeit dargestellt, auf welche Weise der Aromabehälter 10 im Zusammenwirken mit einer Trinkvorrichtung 60 automatisch in eine aktivierte Position gebracht werden kann, wenn durch das Öffnen eines Deckels kein vertikaler Druck von oben mehr auf den Aromabehälter 10 wirkt. Dazu ist im Bereich des Kopfteils 80 der Trinkvorrichtung ein elastisches Element, im vorliegenden Fall eine Schraubenfeder 82, vorgesehen, die den Aromabehälter 10 vertikal nach oben vorspannt. In der Darstellung nach Fig. 23 ist der Aromabehälter nicht in der aktivierten Position, sondern beim Aufsetzen des Aromabehälters auf das Kopfteil 80 der Trinkvorrichtung dargestellt, damit die Schraubenfeder 82 sichtbar ist. Im Betrieb und insbesondere bei der Bewegung des Aromabehälters zwischen einer nicht aktivierten Position und einer aktivierten Position ist es ausreichend, dass die Schraubenfeder 82 den Aromabehälter lediglich eine geringe Wegstrecke nach oben bewegt, wo der Aromabehälter gegen einen Anschlag an demjenigen Teil des Kopfteils 80 anstößt, der sich durch den die inneren Seitenwand 16 gebildeten Raum erstreckt. Auf diese Weise wird beim Abschrauben des Deckels durch die elastische Vorspannung der Schraubenfeder 82 der Aromabehälter eine definierte Wegstrecke nach oben gegen einen Anschlag bewegt, wo er sich in der aktivierten Position befindet. In gleicher Weise wird bei dem Öffnen des Deckels Druck auf die obere Wand 12 des Aromabehälters 10 ausgeübt und dieser in vertikaler Richtung nach unten in die nicht aktivierte Position verschoben.

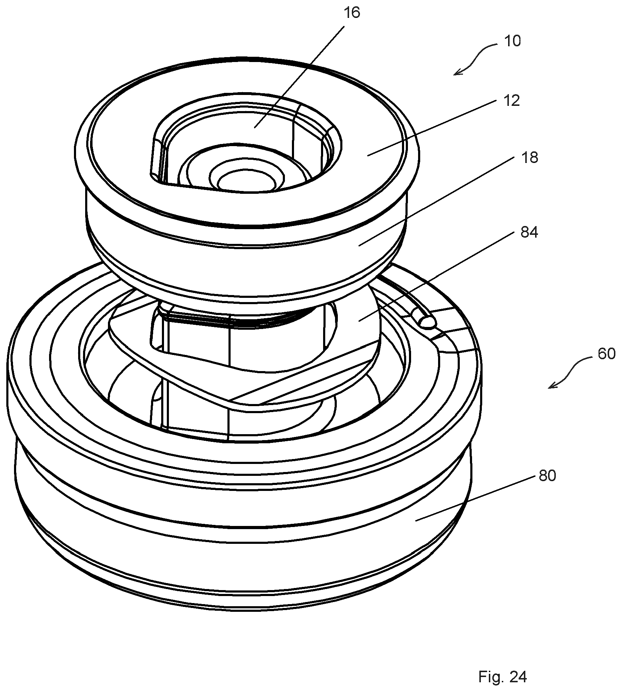

Die Ausführungsform nach den Fig. 24 bis 26 unterscheidet sich von derjenigen nach Fig. 23 lediglich dadurch, dass anstelle der Schraubenfeder 82 ein Federring 84 vorgesehen ist. Der Federring hat gegenüber der Schraubenfeder 82 nach der Ausführungsform nach Fig. 23 den Vorteil, dass dieser leichter zu reinigen ist.

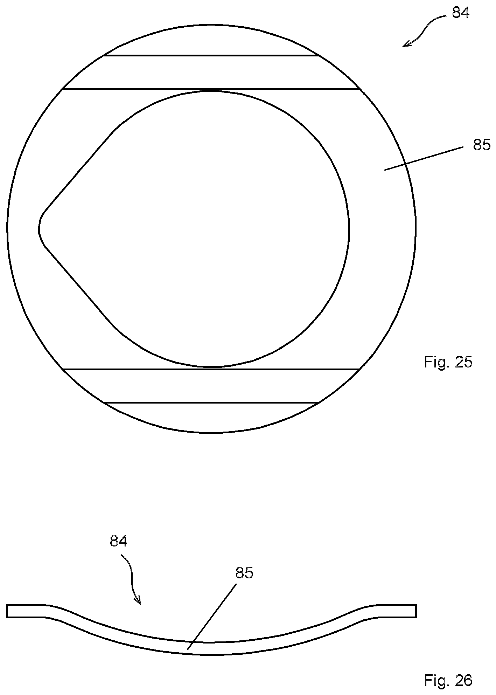

Der Federring kann als separates Bauteil vorgesehen sein, das in eine Vertiefung im Kopfteil 80 der Trinkvorrichtung 60 eingelegt wird und den Aromabehälter 10 von der nicht aktivierten Position vertikal nach oben in die aktivierte Position verschiebt, sobald der Deckel (in Fig. 24 nicht dargestellt) der Trinkvorrichtung abgenommen wird und keinen Druck mehr auf die obere Wand 12 des Aromabehälters ausübt. Eine exemplarische Form des Federrings 24 ist dabei in den Fig. 25 und 26 gezeigt, aus denen ersichtlich ist, dass der Federring lediglich eine Ringplatte ist mit einem gewölbten, elastisch verformbaren Mittelteil 85, die von einem Benutzer leicht gereinigt werden kann.

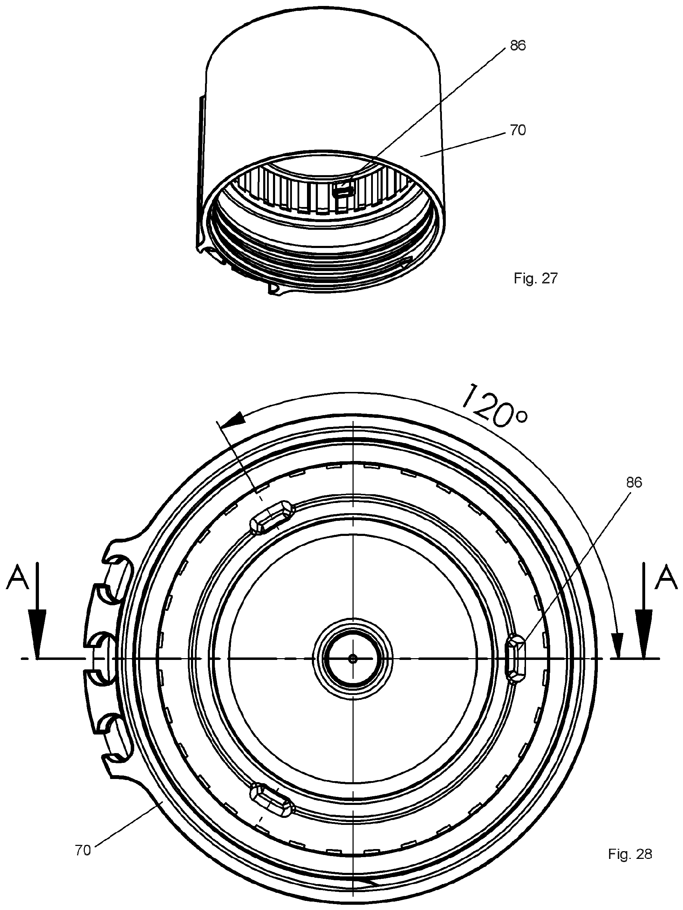

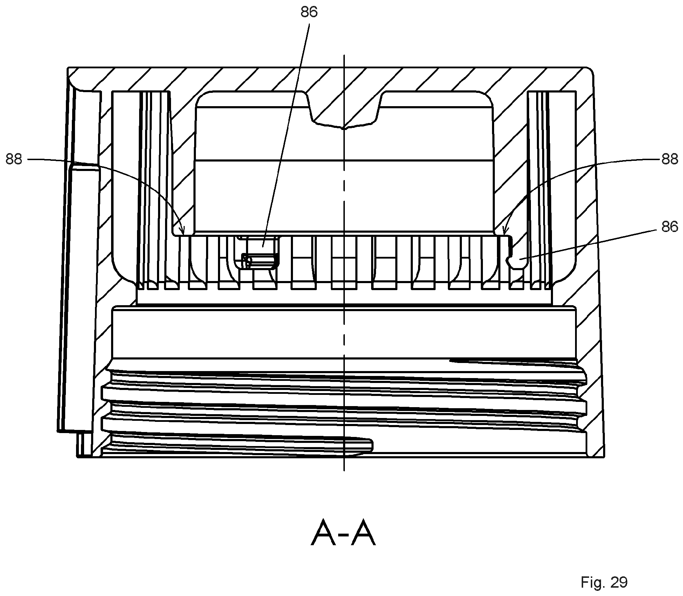

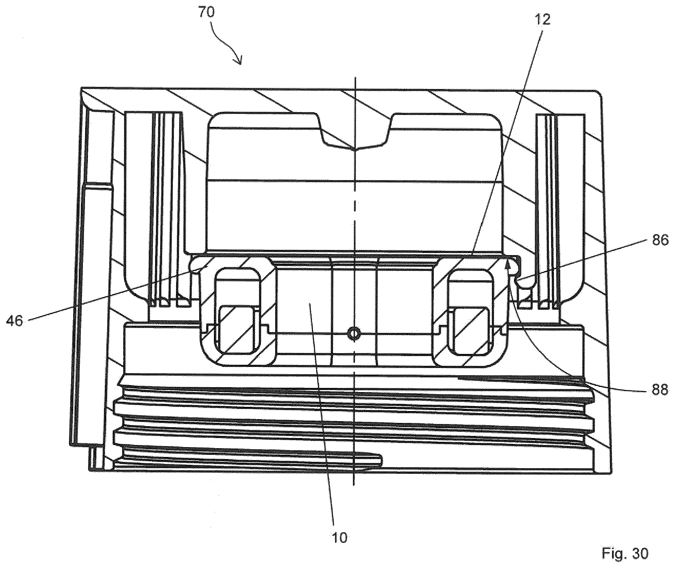

In den Fig. 27 bis 30 sind der Deckel 70 und dessen Zusammenwirken mit einem Aromabehälter 10 nach der Ausführungsform der Fig. 5 bis 8 dargestellt, der ein selbsttätiges Bewegen des Aromabehälters zwischen der nicht aktivierten Position und der aktivierten Position ermöglicht. Der Deckel 70 der Trinkvorrichtung ist dabei mit mindestens einem Eingriffshaken, im Ausführungsbeispiel nach den Fig. 27 bis 30 mit drei Eingriffshaken 86 versehen, die, wie am besten aus der Darstellung der Fig. 28 ersichtlich ist, gleichmäßig am Umfang einer Innenfläche des Deckels 70 verteilt sind. Aus der Schnittdarstellung in Fig. 29 ist dabei die Geometrie eines Eingriffshakens 86 im Schnitt zu erkennen. Die Eingriffshaken erstrecken sich dabei von Anschlagfläche 88 im Deckel nach unten. Die Anschlagfläche 88 besitzt dabei die Funktion, die obere Wand 12 des Aromabehälters 10 zu positionieren. Der Aromabehälter ist mit einem umlaufenden Rand 46 versehen, um den, wie in Fig. 30 dargestellt ist, die Eingriffshaken 86 formschlüssig greifen können. Der Aromabehälter 10 ist auf dem Kopfteil der Trinkvorrichtung exakt in Bezug auf den Winkel positioniert, so dass der Aromabehälter bei einer Drehung des Deckels nicht rotiert, sondern die Eingriffshaken 86 bei einer Drehung des Deckels um den umlaufenden Rand 46 herumbewegt werden und bei dem Abschrauben des Deckels nach oben der Aromabehälter 10 mit nach oben ziehen.

Beim Abschrauben des Deckels erfolgt das nach oben Ziehen des Aromabehälters, bis dieser gegen einen Anschlag stößt. Sobald der Aromabehälter bis in seine aktivierte Position, d.h. seine Betriebsposition, nach oben gezogen wurde und gegen den Anschlag am Mundstück der Trinkvorrichtung (nicht dargestellt) stößt, verformen sich die Eingriffshaken bei einer weiteren vertikalen Bewegung des Deckels 70 nach oben und kommen dabei aus dem Eingriff mit dem umlaufenden Rand 46 des Aromabehälters.

Wird nach der Benutzung der Trinkvorrichtung, während der sich der Aromabehälter 10 in der nach oben gezogenen, aktivierten Position befindet, der Deckel wieder geschlossen, so schnappen die Eingriffshaken 86 über den umlaufenden Rand 46 des Aromabehälters. Die obere Wand 12 des Deckels 70 steht dann gegen die Anschlagfläche 88 des Deckels 70 an, so dass bei einer weiteren vertikalen Bewegung des Deckels nach unten, beispielsweise während des Aufschraubens des Deckels auf ein Gewinde am Kopfteil der Trinkvorrichtung, der Aromabehälter vertikal nach unten gedrückt wird, bis sich der Aromabehälter in der nicht aktivierten Stellung befindet, in der die Austrittsöffnung nicht mehr mit einer entsprechenden Eintrittsöffnung in den Transportkanal für Trinkflüssigkeit in Strömungsverbindung ist und die an der Unterseite des Aromabehälters angeordnet Lufteintrittsöffnung 48 gegen eine abdichtende Fläche am Kopfteil der Trinkvorrichtung gedrückt wird.

Der Benutzer muss somit auch bei dieser Ausgestaltung den Aromabehälter nicht mehr zwischen der nicht aktivierten und der aktivierten Position hin- und herbewegen, da dies selbsttätig beim Abschrauben und Aufschrauben des Deckels erfolgt. Wenn der Benutzer allerdings nicht aromatisierte Trinkflüssigkeit zu sich nehmen möchte, besteht natürlich die Möglichkeit, den Aromabehälter vor dem Trinken vertikal nach unten manuell in die nicht aktivierte Position zu drücken.

Neben den vorangehend beschriebenen Varianten, mit Hilfe derer der Aromabehälter automatisch zwischen der aktivierten und der nicht aktivierten Position bewegt werden kann, sind auch weitere hierin nicht im Detail beschriebene Lösungen denkbar. So kann das Hochziehen des Aromabehälters durch eine magnetische Ankopplung des Aromabehälters mit dem Deckel erfolgen. Anstelle einer Schraubverbindung kann in gleicher Weise auch eine Bajonettverbindung zwischen dem Deckel und dem Trinkbehälter vorgesehen sein.

Ebenfalls ist es möglich, einen Klappdeckel, entweder mit oder ohne Federelement vorzusehen, der beim Öffnen den Aromabehälter entweder freigibt, so dass er durch ein Federelement in die aktivierte Position verschoben werden kann, oder aber mit Hilfe rampenförmiger Schrägflächen am Deckel beim Öffnen und Schließen des Deckels selbsttätig den Aromabehälter geringfügig dreht, um ihn zwischen der aktivierten Position und der nicht aktivierten Position zu bewegen.

Schließlich ist es auch möglich, den Aromabehälter so zu konzipieren, dass er bei Anlegen von Unterdruck selbsttätig von einer nicht aktivierten in eine aktivierte Position bewegbar ist. Bei dieser Lösung ist das bewegte Teil im Aromabehälter ein Rückschlagventil, das im Falle eines Unterdrucks bei der Benutzung der Trinkvorrichtung selbsttätig öffnet und die Luftaustrittsöffnung des Aromabehälters frei gibt.

Allen vorangehend beschriebenen Lösungen ist gemeinsam, dass der Aromabehälter so konzipiert wird, dass die Reynoldszahl $R_e$ im Lufteintrittsbereich größer als 2000 ist, wobei die Reynoldszahl definiert ist als $Re = (w \cdot d)/v$, mit der kinematischen Viskosität von Luft $v$ [m$^2$/s], dem Durchmesser $d$ [m] der Lufteintrittsöffnung und der Strömungsgeschwindigkeit $w$ [m/s] beim Einströmen in den Aromabehälter mit einem gemittelten Volumenstrom zwischen 250 ml/min und 600 ml/min. Vorzugsweise ist die Geometrie der Aromakammer so gestaltet, dass bei diesem Volumenstrom an den Aromabehälter durchströmender Luft im gesamten Aromabehälter eine turbulente Luftströmung herrscht, wobei die lokale Reynoldszahl, welche den lokalen Strömungszustand an einer bestimmten Stelle im Aromabehälter charakterisiert, anstelle des Durchmessers $d$ der Lufteintrittsöffnung unter Zuhilfenahme des jeweils größten freien Strömungsquerschnitts in dem lokalen Bereich des Aromabehälters gebildet wird.

Die vorangehend beschriebenen Ausführungsformen beschreiben einzelne vorteilhafte Ausgestaltungendes erfindungsgemäßen Aromabehälters, die soweit miteinander sinnvoll kombinierbar, auch in weiteren, nicht im Detail beschriebenen Ausführungsformen miteinander kombiniert werden können.

Bezugszeichenliste

10    Aromabehälter
12    obere Wand
14    untere Wand
16    innere Seitenwand
18    äußere Seitenwand
20    obere Schale
22    untere Schale
24    Luftaustrittsöffnung
26    Abflachung
28    spitz zulaufendes Ende
30    Schattenfuge
32    Kopfraum
34    der Aromakammer zugewandte Seite der oberen Wand
36    der Aromakammer zugewandte Seite der unteren Wand

| | |
|---|---|
| 40 | Aromakammer |
| 42 | Trägersubstanz |
| 44 | luftgefüllter Raum der Aromakammer |
| 46 | umlaufender Rand |
| 48 | Lufteintrittsöffnung |
| 50 | Griffmulde |
| 52 | freies Ende des Aromabehälters |
| 54 | Verschlussansatz |
| 55 | Grifffläche |
| 56 | Gleitrippe |
| 58 | Durchgang |
| 60 | Trinkvorrichtung |
| 70 | Deckel |
| 72 | Mitnahmefläche |
| 74 | oberer Randbereich |
| 76 | Mitnahmefläche |
| 78 | oberer Randbereich |
| 80 | Kopfteil |
| 82 | Schraubenfeder |
| 84 | Federring |
| 85 | gewölbtes Mittelteil |
| 86 | Eingriffshaken |
| 88 | Anschlagfläche |

**Patentansprüche**

1. Aromabehälter zur Zugabe einer Aromasubstanz zu einem den Aromabehälter (10) durchströmenden Luftstroms, wobei der Aromabehälter an einer Trinkvorrichtung anbringbar ist und

   - eine obere Wand (12), eine untere Wand (14) sowie mindestens eine Seitenwand (18), die eine Aromakammer (40) umschließen,
   - mindestens eine Lufteintrittsöffnung (48) in die Aromakammer (40); sowie
   - mindestens eine Luftaustrittsöffnung (24) aus der Aromakammer (40) umfasst; wobei
   - sich in der Aromakammer (40) eine Trägersubstanz (42) für die Aromasubstanz befindet;

   **dadurch gekennzeichnet, dass**

   - die Trägersubstanz (42) ein Vliesmaterial umfasst; wobei
   - die Luftdurchlässigkeit L des Vliesmaterials bei 100 Pa Differenzdruck L ≥ 200 l/(m²·s) beträgt, und bevorzugt zwischen 220 l/(m²·s) und 280 1/(m²·s) beträgt;
   - der Aromabehälter weiterhin einen Kopfraum (32) umfasst, der einen nicht mit Trägersubstanz (42) gefüllten Hohlraum in der Aromakammer (40) darstellt; wobei
   - der Kopfraum (32) zwischen der Trägersubstanz (42) und der der Aromakammer (40) zugewandten Seite (34) der oberen Wand (12) vorgesehen ist;
   - sich die mindestens eine Lufteintrittsöffnung (48) im Bereich der unteren Wand (14) des Aromabehälters (10) befindet; und die mindestens eine Luftaustrittsöffnung (24) in einer Seitenwand (16) des Aromabehälters angeordnet ist; und
   - die mindestens eine Lufteintrittsöffnung (48) und die mindestens eine Luftaustrittsöffnung (24) so angeordnet sind, dass eine definierte Wegstrecke des den Aromabehälter durchströmenden Luftstroms den Kopfraum (32) sowie die Trägersubstanz (42) umfasst.

2. Aromabehälter nach Anspruch 1,
   **dadurch gekennzeichnet, dass**
   die Seitenwand (18) zumindest abschnittsweise einen Vorsprung (46) umfasst, der sich vorzugsweise im Wesentlichen senkrecht zur Seitenwand (18) nach außen erstreckt.

3. Aromabehälter nach einem der vorhergehenden Ansprüche, **dadurch gekennzeichnet, dass**
   das Vliesmaterial einen spezifischen Strömungswiderstand von weniger als 500 Pa·s/m und bevorzugt weniger als 400 Pa·s/m und am meisten bevorzugt von etwa 380 Pa·s/m besitzt.

4.  Aromabehälter nach einem der vorherhegenden Ansprüche, **dadurch gekennzeichnet, dass**

    - das Vliesmaterial ein Flächengewicht von weniger als 1500 g/m$^2$ und bevorzugt von etwa 1000 g/m$^2$ besitzt; und
    - das Vliesmaterial eine Dichte von weniger als 300 kg/m$^3$ und bevorzugt eine Dichte von etwa 200 kg/m$^3$ besitzt.

5.  Aromabehälter nach einem der vorhergehenden Ansprüche, **dadurch gekennzeichnet, dass** die Dicke der Trägersubstanz (42) mindestens 50% der Höhe zwischen der oberen Wand (12) des Aromabehälters (10) und der unteren Wand (14) des Aromabehälters (10) und bevorzugt mindestens 80% der Höhe zwischen der oberen Wand (12) des Aromabehälters (10) und der unteren Wand (14) des Aromabehälters (10) beträgt.

6.  Aromabehälter nach einem der vorhergehenden Ansprüche, **dadurch gekennzeichnet, dass** die mindestens eine Lufteintrittsöffnung (48) einen Durchmesser von mindestens 0.2 mm oder eine unterschiedliche Geometrie mit äquivalentem minimalen Öffnungsquerschnitt besitzt, und bevorzugt die mindestens eine Lufteintrittsöffnung (48) einen Durchmesser von nicht mehr als 20 mm besitzt.

7.  Aromabehälter nach einem der vorhergehenden Ansprüche, **dadurch gekennzeichnet, dass** Porosität des Vliesmaterials zwischen 70% und 93% liegt, und bevorzugt zwischen 70% und 80% liegt.

8.  Aromabehälter nach einem der vorhergehenden Ansprüche, **dadurch gekennzeichnet, dass**

    die Reynoldszahl Re im Lufteintrittsbereich des Aromabehälters (10) größer als 2000 ist; wobei
    die Reynoldszahl definiert ist als Re = (w·d)/v, mit
    der kinematischen Viskosität von Luft v [m$^2$/s], dem Durchmesser d [m] der Lufteintrittsöffnung und der Strömungsgeschwindigkeit w [m/s] beim Einströmen in den Aromabehälter mit einem gemittelten Volumenstrom zwischen 250 ml/min und 600 ml/min.

9.  Aromabehälter nach einem der vorhergehenden Ansprüche, **dadurch gekennzeichnet, dass** der Aromabehälter eine im Wesentlichen ringförmige Geometrie aufweist mit einer äußeren Seitenwand (18) und einer inneren Seitenwand (16).

10. Aromabehälter nach Anspruch 9, **dadurch gekennzeichnet, dass** die innere Seitenwand (16) einen Raum umschließt, dessen Querschnittsfläche eine von einer kreisrunden Form abweichende Geometrie besitzt.

11. Aromabehälter nach Anspruch 10, **dadurch gekennzeichnet, dass** die Geometrie der inneren Seitenwand (16) nur eine einzige Position des Aromabehälters (10) in Bezug auf seine Rotation im Zusammenwirken mit einem entsprechend geformten Mundstück einer Trinkvorrichtung definiert, wobei vorzugsweise der durch die innere Seitenwand (16) umschlossene Raum eine im Wesentlichen tropfenförmige Querschnittsfläche besitzt.

12. Aromabehälter nach einem der Ansprüche 10 oder 11, **dadurch gekennzeichnet, dass** für das Verhältnis zwischen einer maximalen Erstreckung $L_{max}$ der Querschnittsfläche des von der inneren Seitenwand (16) umschlossenen Raums und der minimalen Erstreckung $L_{min}$ der Querschnittsfläche des von der inneren Seitenwand umschlossenen Raums gilt: $1.05 \leq L_{max}/L_{min} \leq 1.15$; und bevorzugt $L_{max}/L_{min}$ etwa 1.1 beträgt.

13. Aromabehälter nach einem der Ansprüche 10 bis 12, weiter umfassend Gleitrippen (56) im Bereich der inneren Seitenwand (16).

14. Aromabehälter nach einem der vorhergehenden Ansprüche, **dadurch gekennzeichnet, dass** die Luftaustrittsöffnung in einer inneren Seitenwand (16) im Falle einer im Wesentlichen ringförmigen Geometrie des Aromabehälters mit einer äußeren Seitenwand (18) und einer inneren Seitenwand (16).

15. Aromabehälter nach einem der vorhergehenden Ansprüche, **dadurch gekennzeichnet, dass**

der Aromabehälter eine Unterschale (22) und eine mit der Unterschale (22) verbundene Oberschale (20) umfasst, wobei die Luftaustrittsöffnung (24) im Verbindungsbereich zwischen Unterschale (22) und Oberschale (20) angeordnet ist.

**16.** Trinkvorrichtung, umfassend

- einen Aromabehälter (10) nach einem der vorhergehenden Ansprüche; und
- ein Kopfteil (80), mit dem der Aromabehälter (10) so verbindbar ist, dass zumindest ein Teil des Aromabehälters (10) von einer aktivierten Position in eine nicht aktivierte Position bewegbar ist; wobei
- in der aktivierten Position die Luftaustrittsöffnung (24) in Strömungsverbindung mit einem Transportkanal für Trinkflüssigkeit in der Trinkvorrichtung (60) steht; und
- in der nicht aktivierten Position keine Strömungsverbindung zwischen der Luftaustrittsöffnung (24) und dem Transportkanal für Trinkflüssigkeit besteht, und die Lufteintrittsöffnung (48) vorzugsweise im Wesentlichen abgedichtet ist.

**17.** Trinkvorrichtung nach Anspruch 16,
**dadurch gekennzeichnet, dass**

- die Trinkvorrichtung einen abnehmbaren Deckel (70) umfasst, der auf dem Kopfteil (80) der Trinkvorrichtung (60) anbringbar ist; und
- die Trinkvorrichtung (60) mindestens ein kraftausübendes Element (72; 76; 82; 84; 86) umfasst, mit dem der Aromabehälter (10) beim Abnehmen des Deckels (70) von der nicht aktivierten Position in die aktivierte Position bewegbar ist.

**18.** Trinkvorrichtung nach Anspruch 17,
**dadurch gekennzeichnet, dass**
das mindestens eine kraftausübende Element eine Anschlagfläche (72, 76) am Deckel (70) umfasst, mit welcher der Aromabehälter (10) bei einer Rotation des Deckels (70) beim Abschrauben des Deckels (70) von der nicht aktivierten Position in die aktivierte Position drehbar ist.

**19.** Trinkvorrichtung nach Anspruch 17,
**dadurch gekennzeichnet, dass**

- die Seitenwand (18) des Aromabehälters (10) zumindest abschnittsweise einen Vorsprung (46) umfasst, der sich nach außen erstreckt; und
- das mindestens eine kraftausübende Element ein hakenförmiges Element (86) am Deckel (70) umfasst, das angeordnet und gestaltet ist, um den Vorsprung (46) während des Abnehmens des Deckels formschlüssig zu umgreifen und den Aromabehälter (10) von der nicht aktivierten Position in die aktivierte Position zu bewegen.

**20.** Trinkvorrichtung nach Anspruch 17,
**dadurch gekennzeichnet, dass**
das kraftausübende Element ein elastisches Vorspannelement (82; 84) umfasst, das zwischen dem Aromabehälter (10) und dem Kopfteil (80) der Trinkvorrichtung (60) angeordnet ist und den Aromabehälter (10) bei abgenommenem Deckel in die aktivierte Position vorspannt.

**Claims**

**1.** Aroma container for adding an aroma substance to an air flow flowing through the aroma container (10), wherein the aroma container can be attached to a drinking device, and

- an upper wall (12), a lower wall (14) and at least one side wall (18) enclosing an aroma chamber (40),
- at least one air inlet opening (48) into the aroma chamber (40); as well as
- at least one air outlet opening (24) from the aroma chamber (40); wherein
- there is a carrier substance (42) for the aroma substance in the aroma chamber (40);

**characterised in that**

- the carrier substance (42) comprises a nonwoven material; wherein

- the air permeability L of the nonwoven material at 100 Pa differential pressure is L > 200 1/(m$^2$•s), and preferably between 220 1/(m$^2$•s) and 280 l/(m$^2$•s);

- the aroma container further comprises a headspace (32) which represents a cavity in the aroma chamber (40) that is not filled with carrier substance (42); wherein

- the headspace (32) is provided between the carrier substance (42) and the side (34) of the upper wall (12) facing the aroma chamber (40);

- the at least one air inlet opening (48) is located in the region of the lower wall (14) of the aroma container (10); and the at least one air outlet opening is arranged in a side wall (16) of the aroma container; and

- the at least one air inlet opening (48) and the at least one air outlet opening (24) are arranged in such a way that a defined path of the air flow flowing through the aroma container comprises the headspace (32) and the carrier substance (42).

2. Aroma container according to claim 1,
**characterised in that**
the side wall (18) comprises, at least in sections, a projection (46) which preferably extends outwards substantially perpendicular to the side wall (18).

3. Aroma container according to one of the preceding claims,
**characterised in that**
the nonwoven material has a specific flow resistance of less than 500 Pa•s/m and preferably less than 400 Pa·s/m and most preferably of around 380 Pa•s/m.

4. Aroma container according to one of the preceding claims, **characterised in that**

- the nonwoven material has a weight per unit area of less than 1500 g/m$^2$ and preferably of around 1000 g/m$^2$; and
- the nonwoven material has a density of less than 300 kg/m$^3$ and preferably a density of around 200 kg/m$^3$.

5. Aroma container according to one of the preceding claims,
**characterised in that**
the thickness of the carrier substance (42) amounts to at least 50% of the height between the upper wall (12) of the aroma container (10) and preferably at least 80% of the height between the upper wall (12) of the aroma container (10) and the lower wall (14) of the aroma container (10).

6. Aroma container according to one of the preceding claims,
**characterised in that**
the at least one air inlet opening (48) has a diameter of at least 0.2 mm or a different geometry with an equivalent minimum opening cross-section, and preferably the at least one air inlet opening (48) has a diameter of not more than 20 mm.

7. Aroma container according to one of the preceding claims,
**characterised in that**
the porosity of the nonwoven material lies between 70% and 93%, and preferably between 70% and 80%.

8. Aroma container according to one of the preceding claims,
**characterised in that**

the Reynolds number Re in the air inlet region of the aroma container (10) is greater than 2000; wherein

the Reynolds number is defined as Re = (w•d)/Ʊ, with
the kinematic viscosity of air U [m$^2$/s], the diameter d [m] of the air inlet opening and the flow velocity w [m/s] when flowing into the aroma container with an average volume flow rate between 250 ml/min and 600 ml/min.

9. Aroma container according to one of the preceding claims,
**characterised in that**
the aroma container has a substantially ring-shaped geometry with an outer side wall (18) and an inner side wall (16).

10. Aroma container according to claim 9,

**characterised in that**
the inner side wall (16) encloses a space whose cross-sectional area has a geometry deviating from a circular shape.

11. Aroma container according to claim 10,
    **characterised in that**
    the geometry of the inner side wall (16) only defines a single position of the aroma container (10) with respect to its rotation in interaction with a correspondingly shaped mouthpiece of a drinking device, wherein preferably the space enclosed by the inner side wall (16) has a substantially teardrop-shaped cross-sectional area.

12. Aroma container according to one one of claims 10 or 11, **characterised in that**
    the relationship between a maximum extension $L_{max}$ of the cross-sectional area of the space enclosed by the inner side wall (16) and the minimum extension $L_{min}$ of the cross-sectional area of the space enclosed by the inner side wall is: $1.05 < L_{max}/L_{min} < 1.15$; and preferably $L_{max}/L_{min}$ is around 1.1.

13. Aroma container according to one one of the claims 10 to 12, further comprising sliding ribs (56) in the region of the inner side wall (16).

14. Aroma container according to one of the preceding claims,
    **characterised in that**
    the air outlet opening in an inner sidewall (16) in the case of a substantially ring-shaped geometry of the aroma container with an outer side wall (18) and an inner side wall (16).

15. Aroma container according to one of the preceding claims,
    **characterised in that**
    the aroma container comprises a lower shell (22) and an upper shell (20) connected to the lower shell (22), wherein the air outlet opening (24) is arranged in the connecting region between the lower shell (22) and the upper shell (20).

16. Drinking device, comprising

    - an aroma container (10) according to one of the preceding claims; and
    - a head part (80) with which the aroma container (10) is connectable in such a way that at least part of the aroma container (10) can be moved from an activated position to a non-activated position; wherein
    - in the activated position, the air outlet opening (24) is in fluid connection with a transport channel for drinking liquid in the drinking device (60); and
    - in the non-activated position, there is no flow connection between the air outlet opening (24) and the transport channel for drinking liquid, and the air inlet opening (48) is preferably substantially sealed.

17. Drinking device according to claim 16,
    **characterised in that**

    - the drinking device comprises a removable lid (70) which can be affixed to the head part (80) of the drinking device (60), and
    - the drinking device (60) comprises at least one force-exerting element (72; 76; 82; 84; 86) by means of which the aroma container (10) can be moved from the non-activated position to the activated position when the lid (70) is removed.

18. Drinking device according to claim 17,
    **characterised in that**
    the at least one force-exerting element comprises a stop surface (72, 76) on the lid (70) with which the aroma container (10) can be rotated from the non-activated position to the activated position when the lid (70) is rotated as the lid (70) is unscrewed.

19. Drinking device according to claim 17,
    **characterised in that**

    - the side wall (18) of the aroma container (10) comprises, at least in sections, a projection (46) which extends outwards; and
    - the at least one force-exerting element comprises a hook-shaped element (86) on the lid (70) which is arranged and designed to grasp the protrusion (46) in a form-fitting manner during removal of the lid and to move the aroma container (10) from the non-activated position to the activated position.

**20.** Drinking device according to claim 17,
**characterised in that**
the force-exerting element comprises an elastic pretensioning element (82; 84) which is arranged between the aroma container (10) and the head part (80) of the drinking device (60) and pre-tensions the aroma container (10) into the activated position when the lid is removed.

**Revendications**

**1.** **Récipient** d'arôme pour ajouter une substance d'arôme à un flux d'air traversant le récipient (10) d'arôme, dans lequel le récipient d'arôme peut être fixé à un dispositif à boire et

- une paroi supérieure (12), une paroi inférieure (14) ainsi qu'au moins une paroi latérale (18) qui entourent une chambre (40) d'arôme,
- au moins une ouverture d'entrée (48) d'air dans la chambre (40) d'arôme ; et
- comprend au moins une ouverture de sortie (24) d'air provenant de la chambre (40) d'arôme ; dans lequel
- une substance porteuse (42) pour la substance d'arôme se trouve dans la chambre (40) d'arôme ;

**caractérisé en ce que**

- la substance porteuse (42) comprend un matériau non tissé ; dans lequel
- la perméabilité à l'air L du matériau non tissé à 100 Pa de pression différentielle est L $\geq$ 200 l/(m$^2$·s), et est de préférence comprise entre 220 l/(m$^2$·s) et 280 l/(m$^2$·s) ;
- le récipient d'arôme comprend en outre un espace de tête (32) qui représente une cavité non remplie de substance porteuse (42) dans la chambre (40) d'arôme ; dans lequel
- l'espace de tête (32) est prévu entre la substance porteuse (42) et le côté (34) de la paroi supérieure (12) orienté vers la chambre (40) d'arôme ;
- l'au moins une ouverture d'entrée (48) d'air se trouve dans la zone de la paroi inférieure (14) du récipient (10) d'arôme ; et l'au moins une ouverture de sortie (24) d'air est disposée dans une paroi latérale (16) du récipient d'arôme ; et
- l'au moins une ouverture d'entrée (48) d'air et l'au moins une ouverture de sortie (24) d'air sont disposées de sorte qu'un trajet défini du flux d'air traversant le récipient d'arôme comprend l'espace de tête (32) ainsi que la substance porteuse (42).

**2.** Récipient d'arôme selon la revendication 1,
**caractérisé en ce que**
la paroi latérale (18) comprend au moins sur certaines sections une saillie (46) qui s'étend de préférence sensiblement perpendiculairement à la paroi latérale (18) vers l'extérieur.

**3.** Récipient d'arôme selon l'une quelconque des revendications précédentes,
**caractérisé en ce que**
le matériau non tissé présente une résistance à l'écoulement spécifique inférieure à 500 Pa·s/m et de préférence inférieure à 400 Pa·s/m et le plus préférentiellement d'environ 380 Pa·s/m.

**4.** Récipient d'arôme selon l'une quelconque des revendications précédentes, **caractérisé en ce que**

- le matériau non tissé présente un poids surfacique inférieur à 1500 g/m$^2$ et de préférence d'environ 1000 g/m$^2$ ; et
- le matériau non tissé présente une densité inférieure à 300 kg/m$^2$ et de préférence une densité d'environ 200 kg/m$^2$.

**5.** Récipient d'arôme selon l'une quelconque des revendications précédentes,
**caractérisé en ce que**
l'épaisseur de la substance porteuse (42) est d'au moins 50 % de la hauteur entre la paroi supérieure (12) du récipient (10) d'arôme et la paroi inférieure (14) du récipient (10) d'arôme et de préférence d'au moins 80 % de la hauteur entre la paroi supérieure (12) du récipient (10) d'arôme et la paroi inférieure (14) du récipient (10) d'arôme.

6. Récipient d'arôme selon l'une quelconque des revendications précédentes,
   **caractérisé en ce que**
   l'au moins une ouverture d'entrée (48) d'air présente un diamètre d'au moins 0,2 mm ou une géométrie différente avec une section d'ouverture minimale équivalente, et de préférence, l'au moins une ouverture d'entrée (48) d'air ne présente pas un diamètre supérieur à 20 mm.

7. Récipient d'arôme selon l'une quelconque des revendications précédentes, **caractérisé en ce que**
   la porosité du matériau non tissé est comprise entre 70 % et 93 %, et est de préférence comprise entre 70 % et 80 %.

8. Récipient d'arôme selon l'une quelconque des revendications précédentes,
   **caractérisé en ce que**

   le nombre Reynolds Re dans la zone d'entrée d'air du récipient (10) d'arôme est supérieur à 2000 ; dans lequel le nombre de Reynolds est défini comme Re = (w·d)/v, avec la viscosité cinématique de l'air v [m$^2$/s], le diamètre d [m] de l'ouverture d'entrée d'air et la vitesse d'écoulement w [m/s] à l'entrée dans le récipient d'arôme avec un débit volumique moyen compris entre 250 ml/min et 600 ml/min.

9. Récipient d'arôme selon l'une quelconque des revendications précédentes,
   **caractérisé en ce que**
   le récipient d'arôme présente une géométrie sensiblement annulaire avec une paroi latérale extérieure (18) et une paroi latérale intérieure (16).

10. Récipient d'arôme selon la revendication 9,
    **caractérisé en ce que**
    la paroi latérale (16) intérieure entoure un espace dont la surface de section transversale présente une géométrie différente d'une forme circulaire.

11. Récipient d'arôme selon la revendication 10,
    **caractérisé en ce que**
    la géométrie de la paroi latérale intérieure (16) ne définit qu'une seule position du récipient (10) d'arôme par rapport à sa rotation en interaction avec un embout de forme correspondante d'un dispositif à boire, dans lequel l'espace entouré par la paroi latérale intérieure (16) présente de préférence une surface de section transversale sensiblement en forme de goutte.

12. Récipient d'arôme selon l'une quelconque des revendications 10 ou 11,
    **caractérisé en ce que**
    pour le rapport entre une extension maximale $L_{max}$ de la surface de section transversale de l'espace entouré par la paroi latérale intérieure (16) et l'extension minimale $L_{min}$ de la surface de section transversale de l'espace entouré par la paroi latérale intérieure s'applique : $1,05 \leq L_{max}/L_{min} \leq 1\text{-}15$ ; et de préférence $L_{max}/L_{min}$ est d'environ 1,1.

13. Récipient d'arôme selon l'une quelconque des revendications 10 à 12,
    comprenant en outre des nervures de glissement (56) dans la zone de la paroi latérale intérieure (16).

14. Récipient d'arôme selon l'une quelconque des revendications précédentes,
    **caractérisé en ce que**
    l'ouverture de sortie d'air dans une paroi latérale intérieure (16) dans le cas d'une géométrie sensiblement annulaire du récipient d'arôme avec une paroi latérale extérieure (18) et une paroi latérale intérieure (16).

15. Récipient d'arôme selon l'une quelconque des revendications précédentes,
    **caractérisé en ce que**
    le récipient d'arôme comprend une coque inférieure (22) et une coque supérieure (20) reliée à la coque inférieure (22), dans lequel l'ouverture de sortie (24) d'air est disposée dans la zone de liaison entre la coque inférieure (22) et la coque supérieure (20).

16. Dispositif à boire, comprenant :

- un récipient (10) d'arôme selon l'une quelconque des revendications précédentes ; et
- une partie de tête (80) à laquelle le récipient (10) d'arôme peut être relié de sorte qu'au moins une partie du récipient (10) d'arôme puisse être déplacée à partir d'une position activée dans une position non activée ; dans lequel

- dans la position activée, l'ouverture de sortie (24) d'air est en liaison d'écoulement avec un canal de transport pour un liquide à boire dans le dispositif à boire (60) ; et
- dans la position non activée, il n'existe pas de liaison d'écoulement entre l'ouverture de sortie (24) d'air et le canal de transport pour un liquide à boire, et l'ouverture d'entrée (48) d'air est de préférence sensiblement rendue étanche.

17. Dispositif à boire selon la revendication 16,
   **caractérisé en ce que**

   - le dispositif à boire comprend un couvercle (70) amovible qui peut être fixé sur la partie de tête (80) du dispositif à boire (60) ; et
   - le dispositif à boire (60) comprend au moins un élément d'application de force (72 ; 76 ; 82 ; 84 ; 86), avec lequel le récipient (10) d'arôme peut être déplacé à partir de la position non activée dans la position activée lors du retrait du couvercle (70).

18. Dispositif à boire selon la revendication 17,
   **caractérisé en ce que**
   l'au moins un élément d'application de force comprend une surface de butée (72, 76) sur le couvercle (70) avec laquelle le récipient (10) d'arôme peut être tourné à partir de la position non activée dans la position activée lors d'une rotation du couvercle (70) lors du dévissage du couvercle (70).

19. Dispositif à boire selon la revendication 17,
   **caractérisé en ce que**

   - la paroi latérale (18) du récipient (10) d'arôme comprend au moins sur certaines sections une saillie (46) qui s'étend vers l'extérieur ; et
   - l'au moins un élément d'application de force comprend un élément en forme de crochet (86) sur le couvercle (70) qui est disposé et conçu pour saisir la saillie (46) par coopération de formes pendant le retrait du couvercle et pour déplacer le récipient (10) d'arôme à partir de la position non activée dans la position activée.

20. Dispositif à boire selon la revendication 17,
   **caractérisé en ce que**
   l'élément d'application de force comprend un élément de précontrainte élastique (82 ; 84) qui est disposé entre le récipient (10) d'arôme et la partie de tête (80) du dispositif à boire (60) et précontraint le récipient (10) d'arôme dans la position activée lorsque le couvercle est retiré.

Fig. 1

Fig. 2

10

24

32

40

14

12

20

42

22

36

A-A

Fig. 3

10

40

42

14

34

12

24

48

44

B-B

Fig. 4

Fig. 5

A-A

Fig. 6

Fig. 7

B-B

Fig. 8

Fig. 9

Fig. 10

Fig. 12

Fig. 11

10

12

18

16

54

24

14

Fig. 13a

10

R

12

18

16

54

14

Fig. 14a

16

18

54

12

Fig. 13b

18

55

16

12

Fig. 14b

Fig. 15

Fig. 16

46
10
24
20
30
22
A

Fig. 17

16
46
20
40
22
40
24
42
42

Fig. 18

46
20
30
22

Fig. 19

58
20
22
24
42

Fig. 20

Fig. 21

Fig. 22

Fig. 23

Fig. 24

84

85

Fig. 25

84

85

Fig. 26

86

70

Fig. 27

120°

86

A

A

70

Fig. 28

A-A

Fig. 29

Fig. 30

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2019016096 A1

- DE 202018000382 U1